# EUROPEAN PATENT APPLICATION

(11) **EP 4 070 819 A1**
(43) Date of publication of application: **12.10.2022**
(21) Application number: 20896431.2
(22) Date of filing: 20.11.2020
(51) Int. Cl.: A61K 45/06, A23L 33/00, A61P 1/14

(54) **COMPOSITION HAVING WHOLESOME PERSONALIZED INTESTINAL FLORA DIVERSITY FUNCTION AND APPLICATION**

(30) Priority: 06.12.2019 CN 201911239497
(71) Applicant: Nii, Keni, Tsukuba, Ibaraki 305-0033 (JP); Wang, Shiyue, Jinhua, Zhejiang 322103 (CN)
(72) Inventor: Nii, Keni, Tsukuba, Ibaraki 305-0033 (JP); Wang, Shiyue, Jinhua, Zhejiang 322103 (CN)
(74) Representative: Gong, Jinping
(86) International application number: PCT/CN2020/130277
(87) International publication number: WO 2021/109879

(57) **Abstract**

Provided is a composition functioning to improve personalized gut microbiota diversity function. The composition includes ingredients for strengthening spleen and invigorating Qi, ingredients for clearing damp and promoting the circulation of blood, and intestinal bacteria substrates. The ingredients for strengthening spleen and invigorating Qi include one or more of *Panax ginseng,* an extract of *Panax ginseng, Panax quiquefolium,* and an extract of *Panax quiquefolium;* the ingredients for clearing damp and promoting the circulation of blood include an ingredient for clearing damp and an ingredient for promoting the circulation of blood, where the ingredient for clearing damp includes one of or a mixture of more of *Wolfiporia cocos* and an extract of *Wolfiporia cocos,* and the ingredient for promoting the circulation of blood includes one of or a mixture of more of nattokinase, natto, and an extract of natto; the intestinal bacteria substrates include water-soluble dietary fiber, water-insoluble dietary fiber, or any combination thereof.

## Description

### TECHNICAL FIELD

The present invention relates to the technical fields of medicaments, health-care products or food compositions, and specifically relates to a composition functioning to improve personalized gut microbiota diversity and an application thereof.

### BACKGROUND

As medical innovation of the post-antibiotic era, one of the most important scientific and technological achievements in the recent 10 years is to explain the mutualism, communalism, or parasitism relationships between gut microbiota and human body, as well as the important role of gut microbiota to maintain the homeostasis of physiological functions of the host, human body more and more clearly. Lots of diseases incapable of being treated by modern medical treatment, including metabolic, immune and mental/neurogenic diseases, are closely related to gut microbiota imbalance.

Gut microbiota makes up the shortages of substances and functions in human body with its diversity via a symbiotic relation with the host, human body. Human body has about 20,000-25,000 genes, and there are at least more than 3 million of genes in the microorganism symbiotic inner and outer surface. In other words, the genes possessed by human body by itself are lower than 1%, while the genes of microorganisms living inside and outside the surface of human body account for more than 99%. Gut microbiota is the largest community, and the vast types of genes provide extensive auxiliary functions for the host human body. The usefulness to human health can be simply generalized into: providing nutrition, helping to defense the invasion of harmful extraneous pathogens, accelerating the maturity of immune system, maintaining normal functions of immune system, helping to maintain the constant state of various physiological functions (including various mental/neural physiological activities), and the like. These progresses are renovating the traditional recognition to the metabolic, immune function, mental/neurogenic diseases in medicine such that the science of gut microbiota is entering into the stage of application and treatment from research level (Schimidt et al., Cell, 2018).

It is regretful that the eco-system of gut microbiota symbiotic to human body is worsening acutely. Led by antibiotics invented during the Second World War, the progress of public health, extensive use of pesticides and chemical drugs, environmental pollution, change of dietary structure and popularization of cesarean result in the crisis in inheritance and maintenance of intestinal bacteria. The diversity of gut microbiota forms complex communities varying from minor to dominant. Minor groups (from phylum to species) may in unfavorable conditions in competition and are poorly resistant to environmental factors. The impairment and even extinction of some strains functions thereof lead to change gut microbiota composition and function, or a shift in their local distribution called a state of dysbiosis. At the same time, the incidence rate of the metabolic, immune and mental/neurogenic diseases rapidly increases in multiples and even several tens of times. For example, for a typical disease caused by metabolic abnormality, type II diabetes, it was estimated that the number of patients with type II diabetes had been up to 114 million in 2017 *(Epidemic and Control Status of Diabetes in China Adults).* For another example, autism is originally a kind of rare disease, but it was shown in the *Development Report 3 on Educational Rehabilitation of Autism in China* that the number of Chinese patients had exceeded 10 million in 2019. Metabolic diseases mainly include: type II diabetes, obesity, cardiovascular diseases, cerebrovascular diseases, dyslipidemia, hypertension, hyperuricemia and the like; the immunologic diseases mainly include: food/drug/pollen allergy, eczema, type-I diabetes, irritable bowel syndrome, inflammatory bowel disease, Crohn's disease, multiple sclerosis, rheumatoid, asthma and cancer; the mental/neurogenic diseases mainly include: autism, depression, bipolar disorder, Parkinson's disease, Alzheimer's disease and the like. These diseases are related to multiple systems, but their common pathological basis may be associated with the imbalanced functions of gut microbiota. A professor of the New York University, Martin J. Blaser, has vividly called these diseases like modern epidemics in the book of *Missing Microbes,* and presents evidences that messing microbiota may related.

Gut microbiota is featured by personalization, and a healthy microecosystem is marked by diversity. Personalization refers to the specificity of intestinal bacteria kept by an individual, and is related to background factors such as, inheritance, culture and environment. Gut microbiota personalization generally forms around 3 years old and maintains the whole life basically. The similarity between two people with closest blood relationship (identical twins) is always lower than 50%. Therefore, gut microbiota is compared to fingerprints by scientists. This is very important. The diversity of gut microbiota can be enhanced and maintained only in the premise of personalized gut microbiota, thus achieving the purpose of maintaining microecological balance.

Beneficial bacteria and harmful bacteria are the concepts prevailing in recent decades. However, the concepts are oversimplified and cannot correctly show the functions of gut microbiota and even will cause misleading. Because the vast majority of intestinal bacteria are double-edged, namely, these intestinal bacteria will help the host in certain conditions, while in other conditions, these intestinal bacteria will damage the host human body. For example, helicobacter pylori can cause gastric ulcer and gastric cancer. However, a large number of research data hint that the disappearance of helicobacter pylori causes substantially increased risk of multiple diseases such as, obesity, asthma, allergy and esophagus cancer (Li J. and Perez-Perez GI, 2018). For another example, *Clostridium difficile* is usually a kind of harmless common bacterium, but when the microecosystem of gut microbiota is out of balance due to antibiotics and the like, the abnormal increase of *Clostridium difficile* will cause pseudomembranous colitis which is a kind of fatal disease. Approximately 29,000 patients died within 30 days of the initial diagnosis of C. difficile. Of those, about 15,000 deaths were estimated to be directly attributable to C. difficile infections, making C. difficile a very important cause of infectious disease death in the United States (Press Release of the United States Department of Health and Human Services, February 25, 2015). Therefore, all gut bacteria are doubled edged, the functional balance of the whole flora is the key to improve and maintain the diversity of gut microbiota. Scientists have begun to note this point, and emphasized the beneficial roles for maintaining health condition, preventing and treating related chronic diseases by maintaining a harmonious microecosystem via the mechanism of interdependence and mutual inhibition of various intestinal bacteria (Cho and Blaser, Nature 2012).

Probiotics are utilized to improve the gut microbiota imbalance, which has a comparatively long history. The usefulness of probiotics is not denied, but there are three major limitations from the aspects of personalization and diversity. Firstly, as allochthonous bacteria, probiotics have been proved by multiple clinical trials to be not colonized inside the intestinal tract (Kristensen et al., Genome Med 2016). Secondly, the types of probiotics are limited, incapable of satisfying the requirements of diversity. Thirdly, as allochthonous bacteria, probiotics are competitors to the resident bacteria. Probiotics can hinder the rehabilitation of resident flora to be dangerous factors to break the balance of resident flora (Suez J, et al., Cell, 2018).

Lots of intestinal bacteria ferment ingredients incapable of being digested and absorbed by human body as own nutritional resources, such kind of nutritional resources are called prebiotics. Due to providing nutrients for inherent intestinal bacteria of an individual, prebiotics are theoretically superior to probiotics in the aspects of improving and maintaining personalized gut microbiota. However, up to now, studies are generally aimed at the increase of some specific beneficial bacteria, or decrease of harmful bacteria. Currently, there is lack of studies on the influences of prebiotics on the overall structure functions (Sanders ME et al., Nature Rev. 2019).

The present invention is coordinated with traditional Chinese herbs for strengthening spleen and invigorating Qi, clearing damp and promoting the circulation of blood based on traditional Chinese medicine acquired constitution theory, in combination with various kinds of gut microbiota's substrates to improve and maintain the personalization and diversity of gut microbiota, thereby solving the existing problems above.

According to retrieval and survey, there is no similar patent literature or paper. Related patent literatures surveyed in Chinese mainland and Taiwan districts are as follows: CN201510036300, CN201510344591, CN201510417997, Cn201510471077, CN201610189936, CN201810722697, CN_105505992_A, CN_105532904_A, CN_105535580_A, CN_105641521_A, CN_105670047, CN_105685868_A, CN_105707628_A, CN_105749099_A, CN_105797003_A, CN_106036856_A, CN106943423A, CN_107115364_A, CN_107468716_A, CN_107495049_A, CN_108159090_A, CN_108159201_A, CN_108685005_A, CN_108795823, CN_109988725_A, TW_201641021_A, TW_201726152_A, TW_201914432_A, CN201711033077, CN201810719798, CN_105596445_A, CN_105962086_A, CN_105779343_A, CN_105962086_A, CN_105969680_A, TW_201703778_A, TW_201914596_A, CN108741036A, CN106942724A, CN110214816A, CN108077912A.

### SUMMARY

The present invention relates to an intervention plan to improve and maintain personalized gut microbiota diversity by utilizing the theory of the foundation of acquired constitution from the traditional Chinese medicine, a composition functioning to improve personalized gut microbiota diversity formulated according to the intervention plan, as well as a medicament or food prepared by the composition.

The present invention proves the usefulness thereof to personalized gut microbiota diversity by the gene analysis technology of gut microbiota first. In the recent decades, the progress of high through-put sequencing technology has provided means for the intestinal bacteria gene analysis at molecular level, and prediction of functional and metabolic pathways. The species structure of a community can be known precisely by 16S/ITS sequencing. 16S rRNA gene is a kind of gene to encode ribosomal small subunits of prokaryote, and is the most common and most useful sign in the studies of bacterial systematics. The gene sequence includes 9 variable regions and 10 conserved regions; the conserved region sequence reflects the genetic relationship between species; the variable region sequence reflects the difference between species. The present invention performs amplification and sequencing on the variable region v4 for Operational Taxonomic Units (OTUs) annotation. PICRUSt is a tool which is developed at the earliest and used for predicting microflora functions based on the 16SrRNA gene sequence. After analyzing the composition of the gene function of the existing sequencing microbial genomes, the composition of the functional gene in a sample can be predicted by the composition of the species obtained by 16s sequencing. Different levels (1-3) of prediction can be obtained by the PICRUSt analysis based on 16SrDNA sequencing.

The gene analysis technology of gut microbiota makes significant progress, but the technology remains lots to be uncovered. The present invention is coordinated with the analysis on the system phenotype level, which can make up the limitation of the gene analysis technology. Bristol Stool Scale is utilized in the invention as a physiological parameter for the diversity and health status of gut microbiota. The scientific grounds are as follows: firstly, the nomenclator of probiotics, professor Tomotari Mitsuoka of Riken Institute has pointed that to observe the shape of feces and smell the undesirable odor of feces are the best indicators to judge whether gut microbiota is in health state (Professor Tomotari Mitsuoka interview article, 2015). Secondly, Bristol Stool Scale is considered to be the optimal integrated index to reflect the diversity of gut microbiota based on the analysis on one of the largest human study (Falony et al. Science 2016).

To further prove the improvement effect of the composition on body function by improving gut microbiota diversity, some representative laboratory detection indexes to some related diseases are tested in the present invention. These indexes include: improvement effects on dyslipidemia (serum total cholesterol, serum triglyceride, serum low density lipoprotein cholesterol, serum high density lipoprotein cholesterol), improvement effects on pathoglycemia (fasting blood-glucose and glucose tolerance test), improvement effects on hyperuricemia (serum uric acid), improvement effects on renal function indexes (serum creatinine, estimated glomerular filtration rate), and improvement effects on liver function indexes (serum alanine aminotransferase and serum aspartate aminotransferase).

The core of the present invention is achieving the balanced overall functions of gut microbiota by improving and maintaining the personalized gut microbiota diversity, beyond the restriction to the increase or decrease of an individual strain and the concept of useful bacteria and harmful bacteria. From the three levels of molecule, function and system, the present invention proves the usefulness of the composition in improving and maintaining personalized gut microbiota diversity via two-way adjusting effects. These effects can assist human body to achieve the purpose of strengthening the foundation of acquired constitution based on traditional Chinese medicine, namely, helping the development and growth of human body, maintaining the homeostasis of physiology system after being an adult, prevention, or treatment or adjunctive treatment of gut microbiota imbalance-related diseases. By the detection on the representative laboratory parameters, the composition of the present invention is proved in its 4th level, namely, pathological level of its beneficial effect on preventing and treating related diseases, in particular to metabolic diseases, immune system diseases, dermatological diseases, mental/neurogenic diseases by improving and maintaining the personalized gut microbiota diversity.

The present invention is achieved by the following technical solutions: a novel intervention plan is provided; the present invention applies the theory of strengthening the foundation of acquired constitution based on traditional Chinese medicine, chooses and uses traditional Chinese herbs for strengthening spleen and invigorating Qi, clearing damp and promoting the circulation of blood as well as compatible with substrates demanded by personalized gut microbiota diversity to break through the limitations of the concept of harmful bacteria and beneficial bacteria. Moreover, aiming at the functional balance and diversity of the overall gut microbiota, the present invention plays the roles in prevention, treatment, or adjunctive treatment of the diseases caused by dysbiosis. The composition shows definite two-way adjusting effects on gut microbiota and their predicted function, indicating that the composition has good regulating effects on the structure and diversity of gut microbiota. Meanwhile, the composition shows two-way adjusting effects on Bristol Stool Scale, which supports the effectiveness of the gut microbiota diversity from the physiological phenotype level. Through the representative laboratory detection on related diseases, the composition has improvement effects on abnormal of blood glucose, blood lipid, serum uric acid, renal function, and liver function, which further proves the usefulness of improving and maintaining the personalized gut microbiota diversity from pathological levels. To sum up, the present invention proves that the intervention plan and the composition have beneficial effects on improving and maintaining the personalized gut microbiota diversity from four levels of molecule, function, overall human body, and representative diseases.

The present invention is achieved by the following technical solutions: a composition based on the intervention plan, and an application thereof. Compatibility of medicines include traditional Chinese herbs for strengthening spleen and invigorating Qi, clearing damp and promoting the circulation of blood as well as various kinds of gut microbiota substrates (water-soluble and water-insoluble dietary fiber). All the traditional Chinese medicines or extracts of traditional Chinese medicine can be purchased from medical companies or manufacturers; all the products accord with the national quality standards. All the dietary fibers accord with the national food-grade standards.

The present invention provides a composition functioning to improving personalized gut microbiota diversity; the composition may effectively improve the functions of gut microbiota in human body after being taken.

A composition functioning to improve personalized gut microbiota diversity includes ingredients for strengthening spleen and invigorating Qi, ingredients for clearing damp and promoting the circulation of blood, and intestinal bacteria substrates.

Preferably, the ingredients for strengthening spleen and invigorating Qi include but not limited to one or more of *Panax ginseng,* an extract of *Panax ginseng, Panax quiquefolium,* and an extract of *Panax quiquefolium. Panax ginseng,* the extract of *Panax ginseng, Panax quiquefolium* and the extract of *Panax quiquefolium* can reinforce vital energy, invigorate spleen for benefiting lung. The *Panax ginseng* includes crude *Panax ginseng,* or simply pretreated *Panax ginseng* intermediate products, or crushed or ground *Panax ginseng* powders or granules, or the like. The *Panax quiquefolium* includes crude *Panax quiquefolium,* or simply pretreated *Panax quiquefolium* intermediate products, or crushed or ground *Panax quiquefolium* powders or granules, or the like. The extract of *Panax ginseng* is a primary extract extracted from *Panax ginseng* by the existing extraction methods, or a simply pretreated extract; the major active ingredients are ginsenoside, as well as *Panax ginseng* polysaccharides, polypeptides, amino acids, volatile oils, inorganic elements, and the like; the general content is 0.0001-10%, or the like. The extract of *Panax quiquefolium* is a primary extract extracted from *Panax quiquefolium* by the existing extraction methods, or a simply pretreated extract; the major active ingredients are ginsenoside, as well as volatile oils, amino acids, polysaccharides, flavonoids, inorganic elements, and the like; the general content is 0.0001-10%, or the like. The extract *of Panax ginseng* or the extract of *Panax quiquefolium* may be extracted according the existing methods, and also may be selected from the existing products purchased from the market.

Preferably, the ingredients for clearing damp and promoting the circulation of blood include an ingredient for clearing damp and an ingredient for promoting the circulation of blood, where the ingredient for clearing damp includes but not limited to one of or a mixture of more of *Wolfiporia cocos* and an extract of *Wolfiporia cocos;* and the ingredient for promoting the circulation of blood includes but not limited to one of or a mixture of more of nattokinase, natto, and an extract of natto. The *Wolfiporia cocos* includes one or a combination of more of crude *Wolfiporia cocos,* or treated *Wolfiporia cocos* pieces, blocks or slices, and also includes treated *Wolfiporia cocos* powders or granules, or the like. The extract of *Wolfiporia cocos* is total extracts extracted from *Wolfiporia cocos* by the existing extraction methods, or separated extracts, such as, pachymaran, which may be extracted by itself or selected from the products available on the market. Nattokinase may be directly selected from the existing products available on the market, or may be also replaced by natto, a natto powder or a natto extract. The final dose of the required natto, natto powder or natto extract may be determined by the amount of the fibrin degradation unit taken in need. In this solution, nattokinase dissolves thrombus by direct and indirect ways. Nattokinase may stimulate vascular endothelial cells to produce a tissue-type plasminogen activator (t-PA). t-PA activates plasminogen into plasmin to dissolve fibrous proteins, thus dissolving thrombus directly. Meanwhile, prourokinase in human body is activated into urokinase; urokinase and t-PA co-activate plasminogen, thus indirectly dissolving thrombus.

Preferably, the intestinal bacteria substrates include but not limited to a water-soluble dietary fiber, a water-insoluble dietary fiber, or any combination thereof.

The water-soluble dietary fiber includes one or more of konjaku flour, glucomannan, crude laminarin, pectin, alginic acid, glucan, guar gum, inulin and *Pericarpium citri reticulatae;* or one or more of food ingredients or medicinal materials containing the above ingredients. For example, *Pericarpium citri reticulatae* may be selected as replacement or supplementary pectin.

Preferably, the water-insoluble dietary fiber includes one or more of cellulose, hemicellulose, and methyl cellulose in lignin. Cellulose is a kind of macromolecular polysaccharide with linearly connected glucose and contained in plant cell wall. Cellulose molecules aggregate with each other (crystallization) to form microfibers, which is characterized in that adjacent microfibers are in the same direction to form firm binding bundles, insoluble into water. Hemicellulose is generally called water-insoluble polysaccharides contained in plant cell wall besides cellulose. Hemicellulose is characterized in that various saccharides (xylose, mannose, glucose, arabinose and the like) are bound irregularly into a heterogeneous multimer. Lignocellulose is a kind of organic flocculent fibrous matter obtained by processing natural timber. For example, common water-insoluble dietary fiber includes but not limited to *Ganoderma lucidum, Auricularia auricual, Tremella fuciformis, Lentinus edodes, Polyporus, Hericium erinaceus, Rhizoma gastrodiae, Sanghuangporus lonicericola, Wolfiporia cocos, Psyllium* seed husk, and the like; these are representative food or traditional Chinese medicines containing high-quality hemicellulose and fiber. These materials can be purchased from the market or drugstores.

Preferably, the composition further includes one or more of *Siraitia grosvenorii,* an extract of *Siraitia grosvenorii,* almond, an extract of almond, perilla seed, and an extract of perilla seed. *Siraitia grosvenorii* can be not only brought into spleen and large intestine channels, but also serve as a natural and healthy sweetening agent to improve the taste of the composition at the same time. Almond, extract of almond, perilla seed, and an extract of perilla seed may be further added to the composition. The almond dietary fiber has a content greater than 10%, which is rich in unsaturated fatty acids to lubricate the intestines. Perilla seed is rich in unsaturated fatty acids (>50%) and abundant essential amino acids. That is, perilla seed may provide substrates for gut microbiota, and also has the effect of lubricating the intestines and relaxing the bowels.

In the above components, a certain component may have two functions or more, for example, *Wolfiporia cocos* or extract of *Wolfiporia cocos* has the effects of strengthening spleen and clearing damp and thus, serves as a ministerial drug of the composition, and meanwhile, has more than 90% water-insoluble dietary fiber and thus, has adjuvant effects. For example, for psyllium seed husk, the component not only contains water-soluble dietary fiber, but also contains water-insoluble dietary fiber. Therefore, psyllium seed husk may both serve as the water-soluble dietary fiber and the water-insoluble dietary fiber.

As a preferred embodiment, the ingredients for strengthening spleen and invigorating Qi include one or more of *Panax ginseng,* the extract of *Panax ginseng, Panax quiquefolium,* and the extract of *Panax quiquefolium;* the ingredients for clearing damp and promoting the circulation of blood include an ingredient for clearing damp and an ingredient for promoting the circulation of blood, where the ingredient for clearing damp includes but not limited to one of or a mixture of more of *Wolfiporia cocos* and an extract of *Wolfiporia cocos;* and the ingredient for promoting the circulation of blood includes but not limited to one of or a mixture of more of nattokinase natto and an extract of natto; a daily dose mainly consists of:

| | |
|---|---|
| One of or a mixture of more of *Panax ginseng,* the extract of *Panax ginseng, Panax quiquefolium,* and an extract of *Panax quiquefolium* | 0.001-2 g |
| One of or a mixture of more of *Wolfiporia cocos* and the extract of *Wolfiporia cocos* | 0.001-5 g |
| One of or a mixture of more of nattokinase, natto, and the extract of natto | 500-4000 Fu |
| Water-soluble dietary fiber by total weight of the composition | 10%-70% |
| Water-insoluble dietary fiber by total weight of the composition | 10%-70% |

an adding amount of the nattokinase is directly obtained by conversion according to the amount of the active ingredients in need; an adding amount of the natto or the extract of natto is calculated by the fibrin degradation unit of the nattokinase contained therein.

In this present invention, the daily dose may be taken once or twice or multiple times per day.

As a more preferred embodiment, the daily dose mainly consists of:

| | | |
|---|---|---|
| Ingredients for strengthening spleen and invigorating Qi | One of or a mixture of more of *Panax ginseng* and *Panax quiquefolium* | 0.01-2 g |
| | Or, one of or a mixture of more of the extract of *Panax ginseng* and the extract of *Panax quiquefolium* | 0.001-0.2 g |
| Ingredients for clearing damp and promoting the circulation of blood | One of or a mixture of more of *Wolfiporia cocos* and the extract of *Wolfiporia cocos* | 0.001-5 g |
| | One of or a mixture of more of nattokinase, natto, and the extract of natto | 500-4000 Fu |
| Water-soluble dietary fiber by total weight of the composition | | 10%-70% |
| Water-insoluble dietary fiber by total weight of the composition | | 10%-70% |

an adding amount of the nattokinase is directly obtained by conversion according to the amount of the active ingredients in need; an adding amount of the natto or the extract of natto is calculated by the fibrin degradation unit of the nattokinase contained therein;

As a more preferred embodiment, the composition further includes 0.1-3 g (further preferably, 0.3-1.5 g) *Siraitia grosvenorii* or 0.001-0.5 g (further preferably, 0.001-0.2 g) extract of *Siraitia grosvenorii;* or simultaneously or separately includes 0.25-1 g) almond and/or 0.25-1 g perilla seeds. The solution has treatment or relief effects on constipation patients.

In the above components, when *Wolfiporia cocos* is selected as the ingredient for clearing damp, 0.1-5 g crude *Wolfiporia cocos* need to be added; when the extract of *Wolfiporia cocos* is selected, 0.001-2 g extract of *Wolfiporia cocos* need to be added.

The water-soluble dietary fiber and the water-insoluble dietary fiber are further respectively and separately preferred as follows: the water-soluble dietary fiber accounts for 20%-60% by the total dose of the formula; the water-insoluble dietary fiber accounts for 20%-60% by the total dose of the formula. Calculated by the daily dose, the water-soluble dietary fiber or the water-insoluble dietary fiber has an adding amount of 1-10 g.

As a specific preferred embodiment, calculated by the daily dose, 0.1-1.2 g *Panax ginseng* and/or *Panax quiquefolium* may be selected as the ingredient for strengthening spleen and invigorating Qi; or 0.005-0.05 g extract of *Panax ginseng* and/or extract of *Panax quiquefolium* may be selected, or a compound of the above two solutions may be selected.

As a specific preferred embodiment, calculated by the daily dose, 0.2-4 g *Wolfiporia cocos* or 0.001-2 g extract of *Wolfiporia cocos* may be selected as the ingredient for clearing damp; 500-4000 Fu nattokinase or 10-100 g fresh natto may be selected as the ingredient for promoting the circulation of blood; further preferably, 10-50 g, or 5-50 g dry natto powder, or a crude extract of natto may be selected. *Wolfiporia cocos* and/or extract of *Wolfiporia cocos* may be not only used as a ministerial drug to assist the ingredient for strengthening spleen and invigorating Qi, but also may be used as a water-insoluble dietary fiber to have an adjuvant effect.

As a specific preferred embodiment, calculated by the daily dose, the composition includes the following components:

| | |
|---|---|
| One of or a mixture of more of *Panax ginseng* or *Panax quiquefolium* | 0.1-1 g |
| *Wolfiporia cocos* | 0.5-4 g |
| Nattokinase | 500-4000 Fu |
| Guar gum | 0.05-0.4 g |
| Alginic acid | 0.05-0.4 g |
| Crude laminarin | 0.05-0.4 g |
| *Pericarpium citri reticulatae* | 0.05-0.4 g |
| Glucomannan | 0.1-0.8 g |
| *Psyllium* seed husk | 0.25-2 g |
| *Siraitia grosvenorii* | 0.35-1.4 g. |

As a specific preferred embodiment, calculated by the daily dose, the composition includes the following components:
One of or a mixture of two of extract of *Panax ginseng* or extract of *Panax quiquefolium* 0.01-0.04 g

| | |
|---|---|
| *Wolfiporia cocos* | 0.5-4 g |
| Nattokinase | 500-4000 Fu |
| Guar gum | 0.05-0.4 g |
| Alginic acid | 0.05-0.4 g |
| Crude laminarin | 0.05-0.4 g |
| *Pericarpium citri reticulatae* | 0.05-0.4 g |
| Glucomannan | 0.1-0.8 g |
| *Psyllium* seed husk | 0.25-2 g |
| *Siraitia grosvenorii* | 0.35-1.4 g. |

The present invention provides an application of the composition of any one of the above technical solutions in the preparation of a medicament, food or health-care product functioning to improve personalized gut microbiota diversity at the same time.

The present invention provides an application of the composition of any one of the above technical solutions in prevention, or treatment, or adjunctive treatment of an gut microbiota imbalance-related disease.

The present invention provides an intervention plan to improve and maintain the gut microbiota diversity by means of the theory of the foundation of acquired constitution based on traditional Chinese medicine. The plan applies the traditional Chinese herbs for strengthening spleen and clearing damp, and promoting the circulation of blood, and is coordinated with compound intestinal bacteria substrates to improve and maintain the personalized gut microbiota diversity via two-way regulation on the gut microbiota functions.

Preferably, the medicament is a medicament for prevention, or treatment, or adjunctive treatment of an gut microbiota imbalance-related disease. The food or health-care product is a food or health-care product having a function of improving gut microbiota imbalance.

The present invention discloses an application of the composition of any one of the above technical solutions in improving personalized gut microbiota diversity at the same time. For example, the composition may be used in improving personalized gut microbiota diversity in a form of a medicament, food or a health-care product. Further, the present invention provides an application thereof in prevention, or treatment, or adjunctive treatment of an gut microbiota imbalance-related disease.

Preferably, the gut microbiota imbalance includes low diversity of types and functions of gut microbiota.

Preferably, the gut microbiota imbalance includes but not limited to relatively strong types and functional genomes of dominant gut microbiota, and relatively slow types and functional genomes of less abundant gut microbiota.

Preferably, the gut microbiota imbalance-related disease includes one or more of a metabolic disease, an immunologic disease, a disease of skin system, a mental/neurogenic disease and a digestive system disease which are associated with gut microbiota imbalance, as well as a low sex hormone-related disease.

Preferably, the metabolic dysfunction disease includes prediabetes, type-II diabetes, obesity, cardiovascular diseases, cerebrovascular diseases, hyperlipidemia, hypertension, hyperuricemia and renal dysfunction.

The disease caused by hyperuricemia mainly includes but not limited to gout, hyperuricemia-caused acute/chronic arthritis, uric acid-derived kidney stone, uric acid-caused acute or chronic renal dysfunction.

Preferably, the immunologic dysfunction disease includes food/drug/pollen allergy, eczema, type-I diabetes, irritable bowel syndrome, inflammatory bowel disease, Crohn's disease, multiple sclerosis, rheumatoid, chronic fatigue disease, asthma and cancer.

Preferably, the dermatological diseases includes atropine dermatitis, psoriasis, eczema, acne, pachylosis and skin aging which are associated with gut microbiota imbalance.

Preferably, the mental/neurogenic disease includes depression, bipolar disorder, autism, schizophrenia, fibromyalgia, insomnia, Parkinson's disease, and Alzheimer's disease.

Preferably, the digestive system disease includes constipation, diarrhea, fatty liver and liver cirrhosis.

Preferably, the low sex hormone-related disease includes developmental retardation of primary/secondary sex characters of children and adolescents, abnormality and disease associated with female physiological cycle and gestation period, osteoporosis caused by estrogen deficiency of postmenopausal female, male and female sexual hypofunction, and climacteric syndrome.

Preferably, the medicament may improve the body biodegradation to an exogenous toxic substance. The exogenous toxic substance includes pesticides, herbicides, food additives, plastic residues, rubber residues, environmental pollutants, heavy metals, and other hazardous chemical substances.

As a preferred embodiment, the exogenous toxic substance includes one or more of halobenzoic acid, halocyclohexane, halobenzene, caprolactam, 2-chloro-4-ethylamino-6-isopropylamino-1,3,5-triazine, dioxin, aminobenzoate and xylene.

The present invention proves that the composition may inhibit the dominant bacteria and functional genomes thereof, and strengthen the less abundant bacteria and functional genomes thereof, promote the convergence of body phenotype towards the intermediate type, and have good two-way regulation effects on gut microbiota. The composition may play the roles of prevention or therapeutical effects on metabolic, immune function, mental/neurogenic diseases and other gut microbiota imbalance-related diseases by improving and maintaining the diversity of personalized gut microbiota.

### BRIEF DESCRIPTION OF DRAWINGS

FIG. 1 is a Bristol Stool Chart;
FIG. 2a shows negative correlations of relative abundance between before and after administration of each bacterium (at the level of Phylum) (the left is a graphical result of 14-day dosing; the right is a graphical result of 30-day dosing);
FIG. 2b shows negative correlations of relative abundance between before and after administration of each bacterium (at the level of Class) (the left is a graphical result with 14-day dosing; the right is a graphical result with 30-day dosing);
FIG. 2c shows negative correlations of relative abundance between before and after administration of each bacterium (at the level of Order) (the left is a graphical result with 14-day dosing; the right is a graphical result with 30-day dosing);
FIG. 2d shows negative correlations of relative abundance between before and after administration of each bacterium (at the level of Family) (the left is a graphical result with 14-day dosing; the right is a graphical result with 30-day dosing);
FIG. 3 are graphical results showing an inhibitory effect of the solution provided by the examples on the relative abundance of the most dominant bacterium (at the level of Phylum), *Bac_Firmicutes* (the left is a graphical result with 14-day dosing; the right is a graphical result with 30-day dosing);
FIG. 4 are graphical results showing an inhibitory effect of the solution provided by the examples on the relative abundance of the second dominant bacterium (at the level of Phylum), *Bac Bacteroidetes* (the left is a graphical result with 14-day dosing; the right is a graphical result with 30-day dosing);
FIG. 5 are graphical results showing an inhibitory effect of the solution provided by the examples on the relative abundance of the third dominant bacterium (at the level of Phylum), *Bac_Proteobacteria* (the left is a graphical result with 14-day dosing; the right is a graphical result with 30-day dosing);
FIG. 6 is a graphical result showing influences of the solution provided by the examples on the relative abundance of the less abundant bacterium (at the level of Phylum) with 14-day dosing;
FIG. 7 is a graphical result showing influences of the solution provided by the examples on the relative abundance of the less abundant bacterium (at the level of Phylum) with 30-day dosing;
FIG. 8 is a graphical result showing influences of the solution provided by the examples on a *Bc_Firmicutes*/*Bc_Bacteroidetes* ratio with 14-day dosing;
FIGS. 9a-9c are respectively graphical results showing influences of the solution provided by the examples on the relative abundance of *Lachnospriraceae, Parabacteroides* and *Bacteroidetes* with 14-/30-day dosing;
FIG. 10 is a graphical result showing related analysis on the change of the whole predicted metabolic pathways (213 types in total) with 14-/ 30-day dosing and the relative abundance before administration according to the solution provided by the examples;
FIG. 11 shows changes of the relative abundance of cell transformation-related genome with 14-/30-day dosing according to the solution provided by the examples;
FIG. 12 shows changes of the relative abundance of transcription-related genome with 14-/30-day dosing according to the solution provided by the examples;
FIG. 13a shows changes of the relative abundance of cytomembrane transport-related genome with 14-/30-day dosing according to the solution provided by the examples;
FIG. 13b shows changes of the relative abundance of electron transfer carriers-related genome with 14-/30-day dosing according to the solution provided by the examples;
FIG. 13c shows changes of the relative abundance of inorganic ion transport-related genome with 14-/30-day dosing according to the solution provided by the examples;
FIG. 13d shows changes of the relative abundance of pores ion channel-related genome with 14-/30-day dosing according to the solution provided by the examples;
FIG. 14 shows changes of the relative abundance of cell regeneration and repair-related genome with 14-/30-day dosing according to the solution provided by the examples;
FIG. 15 shows changes of the relative abundance of cell growth and death-related genome with 14-/30-day dosing according to the solution provided by the examples;
FIG. 16 shows changes of the relative abundance of apoptosis-related genome with 14-/30-day dosing according to the solution provided by the examples;
FIG. 17 shows changes of the relative abundance of ubiquitin system-related genome with 14-/30-day dosing according to the solution provided by the examples;
FIG. 18 shows changes of the relative abundance of glycan biosynthesis and metabolism with 14-/30-day dosing according to the solution provided by the examples;
FIG. 19 shows changes of the relative abundance of xenobiotics degradation-related genes with 14-/30-day dosing according to the solution provided by the examples;
FIGS. 20a and 20b respectively show changes of the relative abundance of a drug metabolism cytochrome p450 and a xenobiotics metabolism cytochrome p450 with 14-/30-day dosing according to the solution provided by the examples;
FIG. 21a shows changes of the relative abundance of terpenes and polyketones biosynthesis-related genes with 14-/30-day dosing according to the solution provided by the examples;

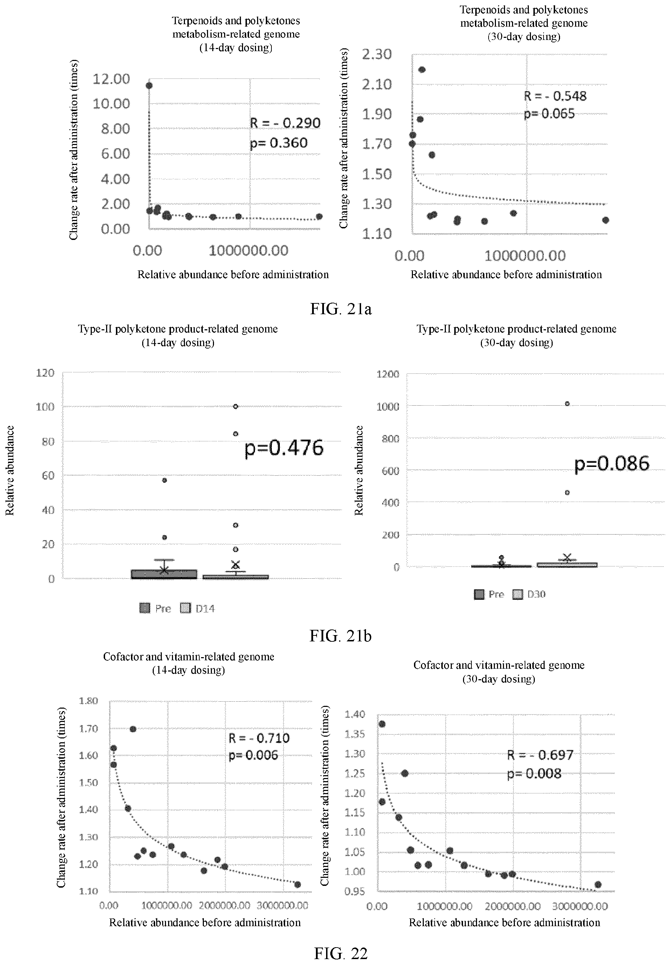

FIG. 21 shows changes of the relative abundance of type-II polyketones biosynthesis-related genes with 14-/30-day dosing according to the solution provided by the examples;
FIG. 22 shows changes of the relative abundance of cofactor and vitamin biosynthesis-related genes with 30-day dosing according to the solution provided by the examples;

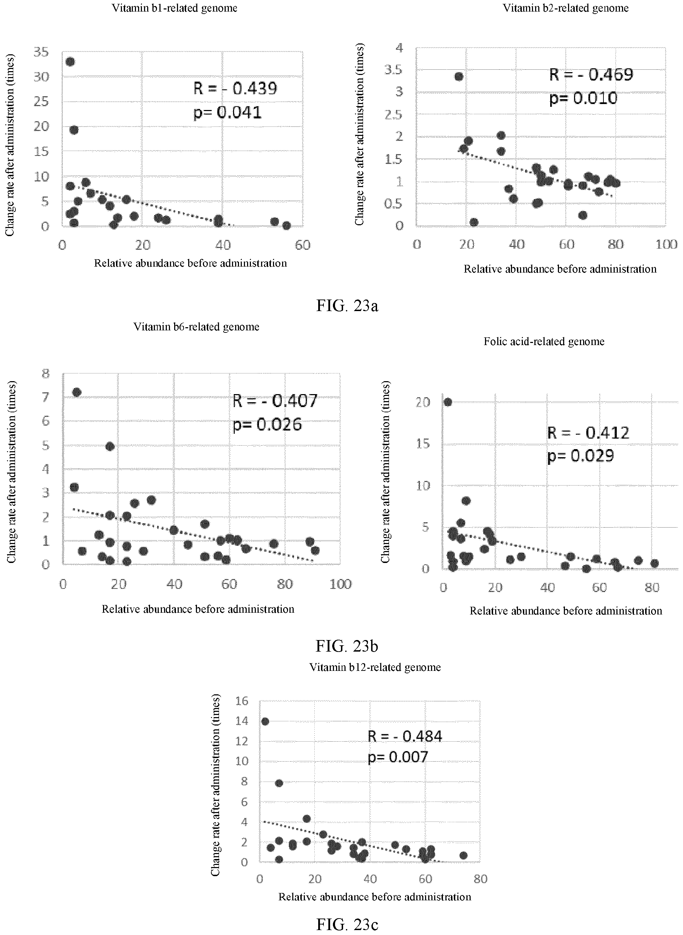

FIG. 23a shows changes of the relative abundance of vitamins b1 and b2 synthesis-related genome with 30-day dosing according to the solution provided by the examples;
FIG. 23b shows changes of the relative abundance of vitamin b6 and folic acid synthesis-related genes with 30-day dosing according to the solution provided by the examples;
FIG. 23c shows changes of the relative abundance of vitamin b12 synthesis-related genes with 30-day dosing according to the solution provided by the examples;

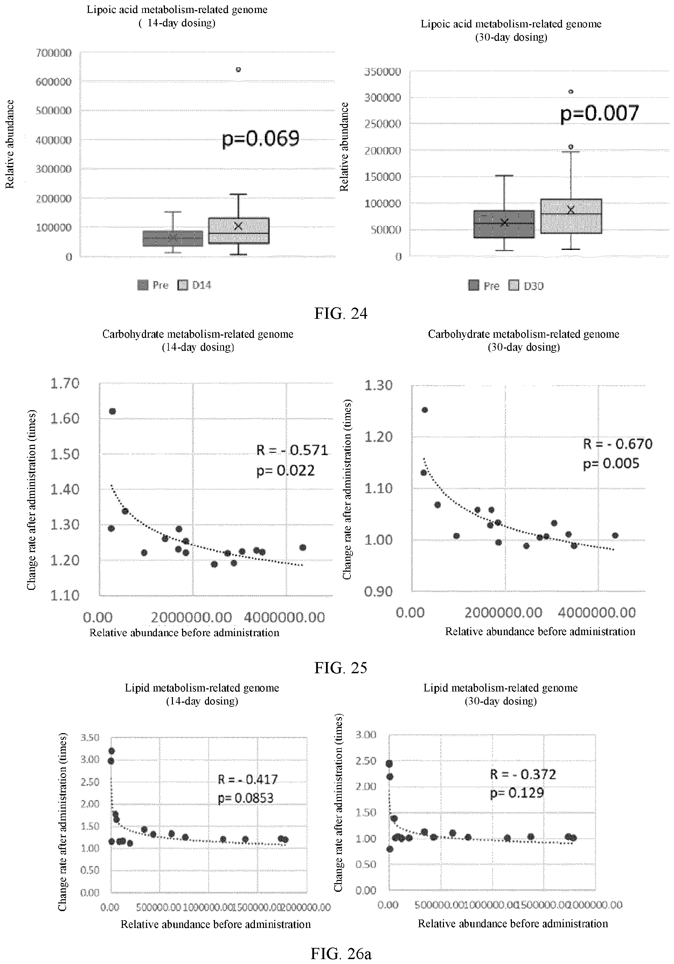

FIG. 24 shows changes of the relative abundance of lipoic acid metabolism-related genes with 14-/30-day dosing according to the solution provided by the examples;
FIG. 25 shows changes of the relative abundance of carbohydrate metabolism-related genes with 14-/30-day dosing according to the solution provided by the examples;
FIG. 26a shows changes of the relative abundance of lipid metabolism-related genes with 14-/30-day dosing according to the solution provided by the examples;

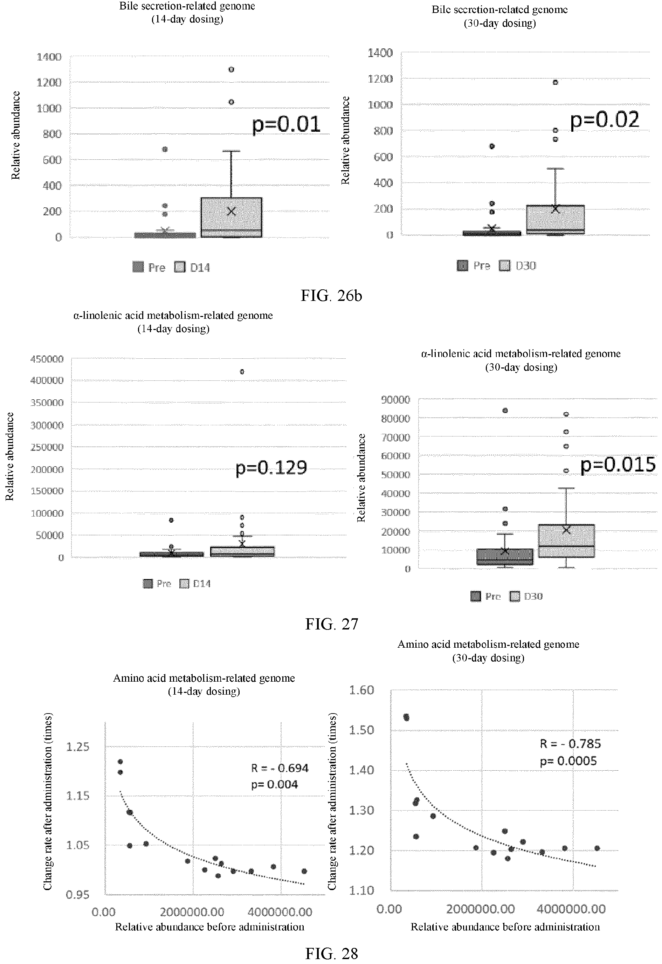

FIG. 26b shows changes of the relative abundance of bile secretion-related genes with 14-/30-day dosing according to the solution provided by the examples;
FIG. 27 shows changes of the relative abundance of α-linolenic acid metabolism-related genes with 14-/30-day dosing according to the solution provided by the examples;
FIG. 28 shows changes of the relative abundance of amino acid metabolism-related genes with 14-/30-day dosing according to the solution provided by the examples;

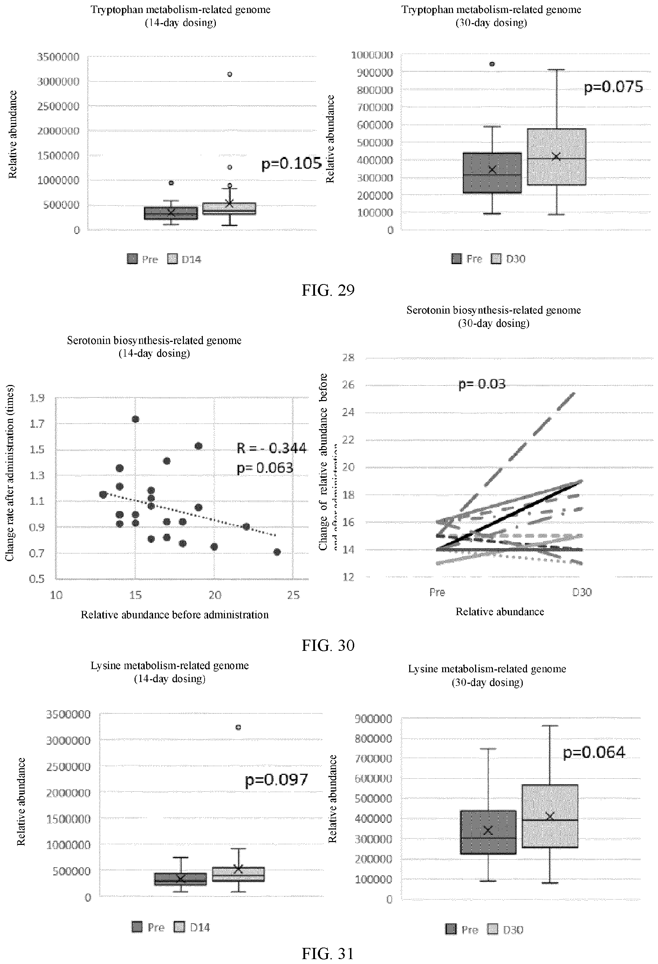

FIG. 29 shows changes of the relative abundance of tryptophan-related genes with 14-/30-day dosing according to the solution provided by the examples;
FIG. 30 (the left) shows changes of the relative abundance of serotonin biosynthesis-related genes with 30-day dosing according to the solution provided by the examples;
FIG. 30 (the right) shows changes of the relative abundance of genes related to the biosynthesis of serotonin in the crowd with weak relative synthetic ability with 14-/30-day dosing according to the solution provided by the examples;
FIG. 31 shows changes of the relative abundance of lysine biosynthesis and metabolism-related genes with 30-day dosing according to the solution provided by the examples;

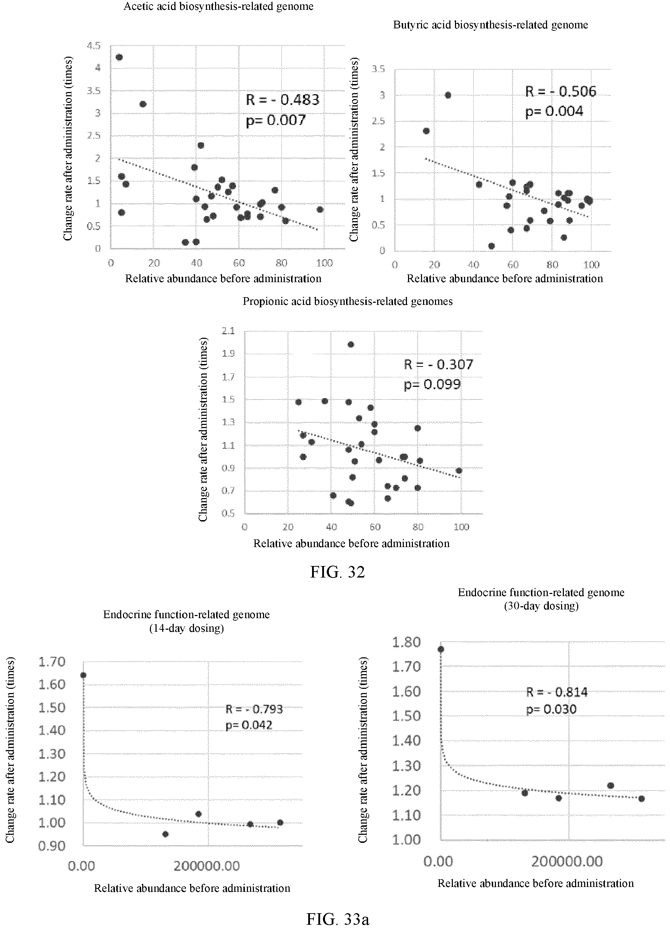

FIG. 32 shows changes of the relative abundance of acetic acid/propionic acid/butyric acid biosynthesis-related genes with 30-day dosing according to the solution provided by the examples;
FIG. 33a shows changes of the relative abundance of endocrine system-related genes with 14-/30-day dosing according to the solution provided by the examples;

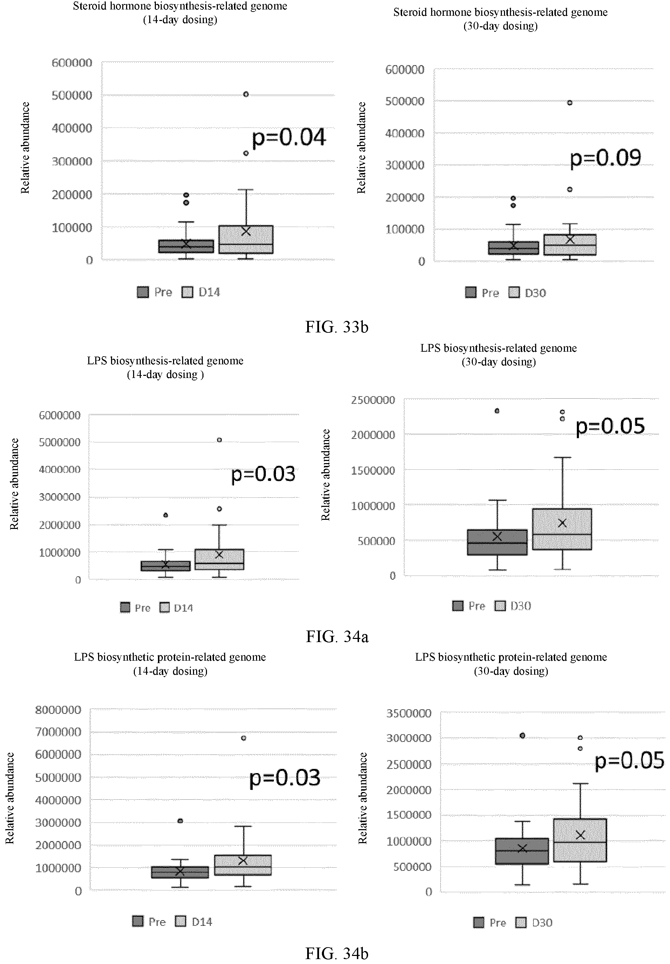

FIG. 33b shows changes of the relative abundance of steroids hormone biosynthesis and metabolism-related genes with 14-/ 30-day dosing according to the solution provided by the examples;
FIG. 34a shows changes of the relative abundance of polysaccharide (LPS) biosynthesis-related genes with 14-/30-day dosing according to the solution provided by the examples;
FIG. 34b shows changes of the relative abundance of LPS biosynthetic protein-related genes with 14-/30-day dosing according to the solution provided by the examples;

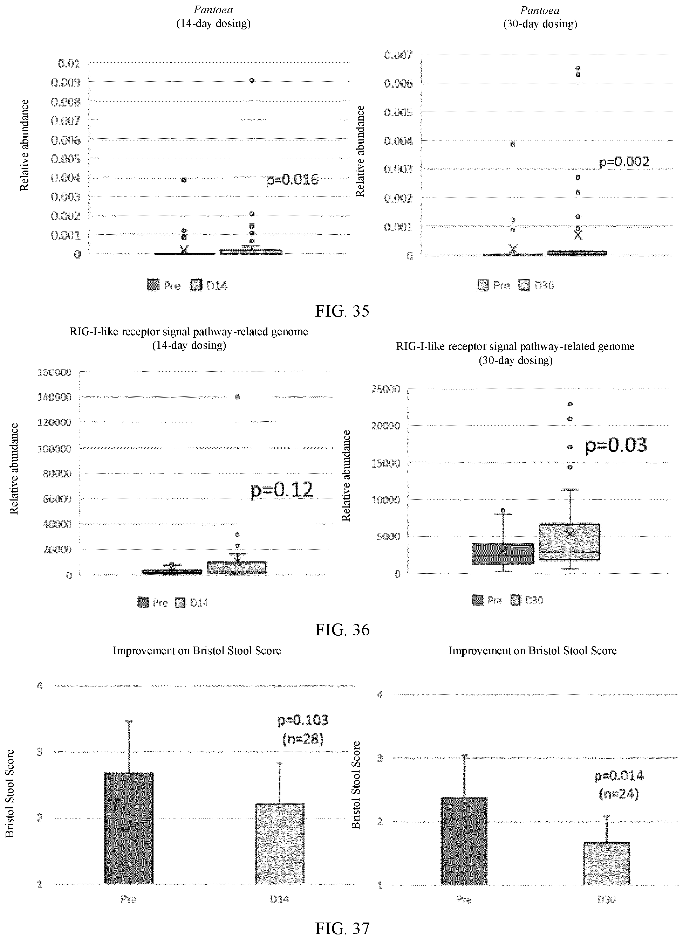

FIG. 35 shows changes of the relative abundance of *Pantoea* gut microbiota with 14-/30-day dosing according to the solution provided by the examples;
FIG. 36 shows changes of the relative abundance of RIG-I-like receptor signal transduction-related genes with 14-/30-day dosing according to the solution provided by the examples;
FIG. 37 shows changes of the Bristol Stool Score with 14-/30-day dosing according to the solution provided by the examples;

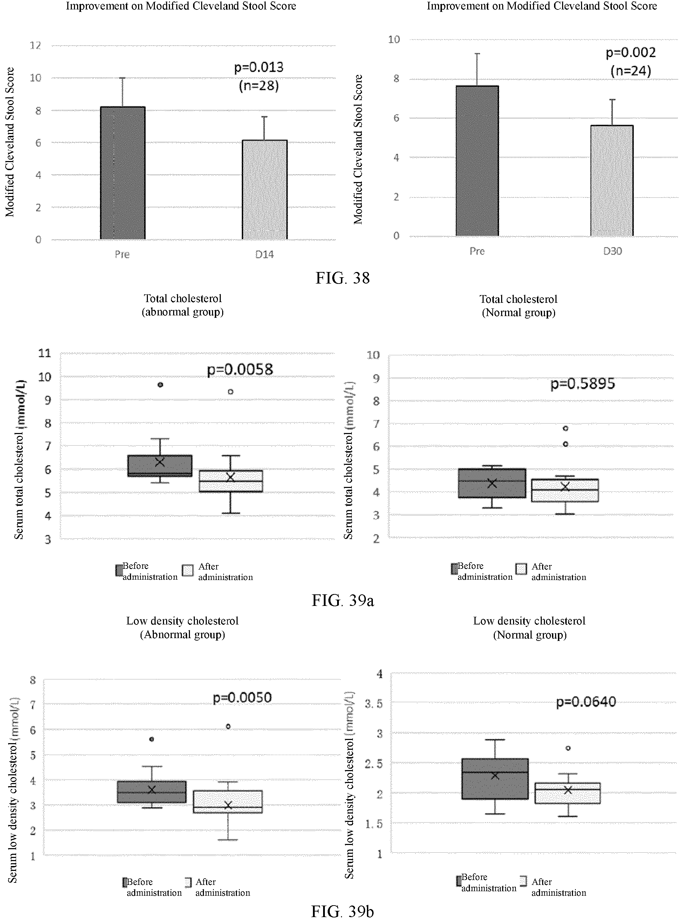

FIG. 38 shows changes of Modified Cleveland Stool Score with 14-/30-day dosing according to the solution provided by the examples;
FIG. 39a shows changes of the serum total cholesterol before and after administration 30-day dosing according to the solution provided by the examples;

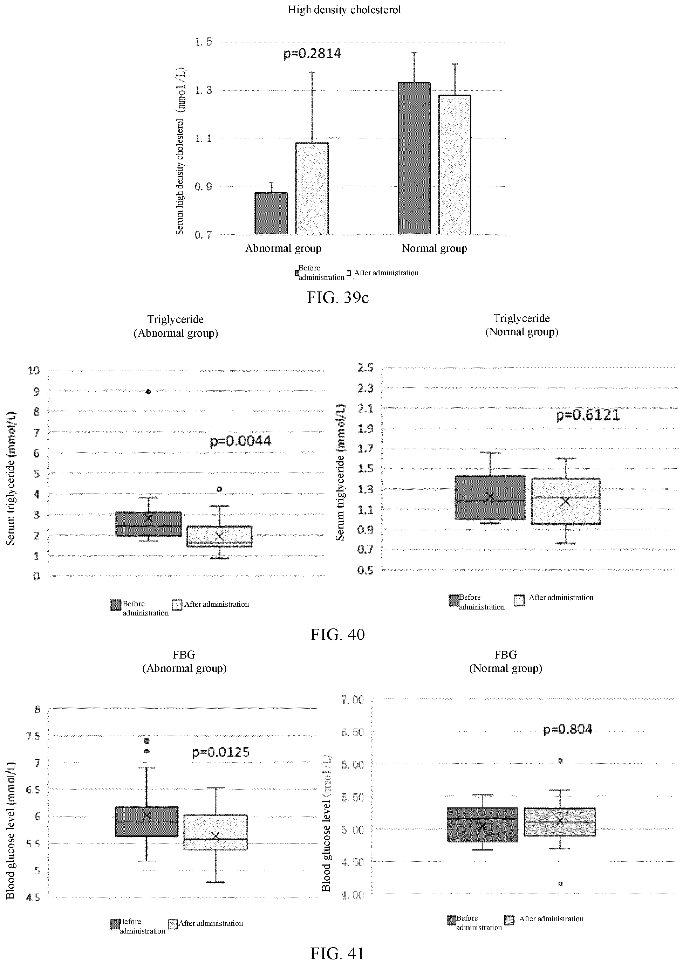

FIG. 39 shows changes of the serum low density lipoprotein cholesterol before and after administration 30-day dosing according to the solution provided by the examples;
FIG. 39 shows changes of the serum high density lipoprotein cholesterol before and after administration 30-day dosing according to the solution provided by the examples;
FIG. 40 shows changes of the serum triglyceride before and after administration 30-day dosing according to the solution provided by the examples;
FIG. 41 shows changes of fasting blood-glucose before and after administration 30-day dosing according to the solution provided by the examples;

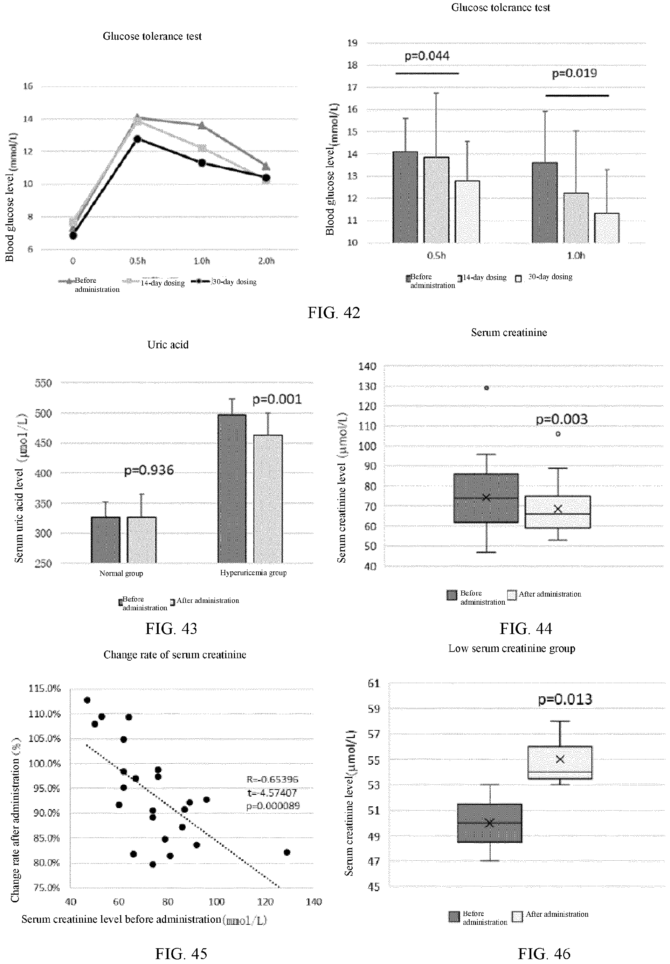

FIG. 42 shows improvement effects on glucose tolerance test with 30-day dosing according to the solution provided by the examples;
FIG. 43 shows changes of serum uric acid before and after administration 30-day dosing according to the solution provided by the examples;
FIG. 44 shows changes of serum creatinine with 30-day dosing according to the solution provided by the examples;
FIG. 45 shows a correlation between the concentration of serum creatinine before and after 30-day dosing according to the solution provided by the examples;
FIG. 46 shows changes of serum creatinine in volunteers with low serum creatinine before and after 30-day dosing according to the solution provided by the examples;

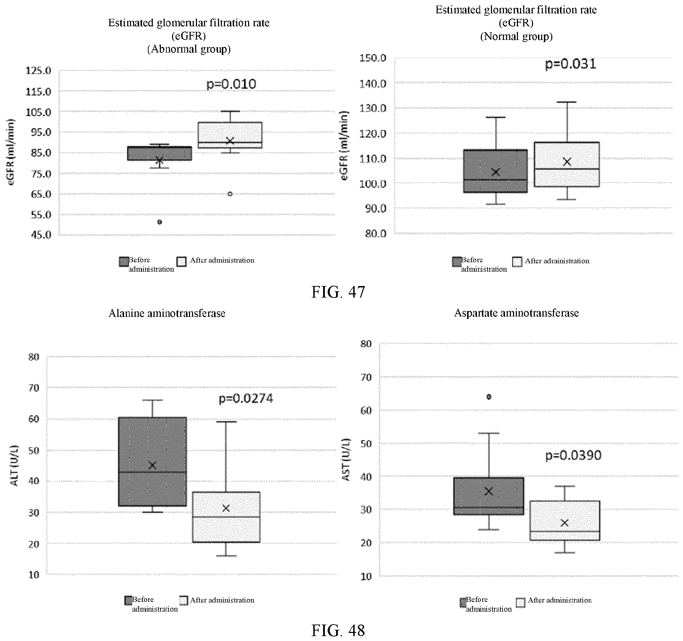

FIG. 47 shows improvement on the estimated glomerular filtration rate before and after 30-day dosing according to the solution provided by the examples;
FIG. 48 shows improvement on the alanine aminotransferase and aspartate aminotransferase before and after 30-day dosing according to the solution provided by the examples.

### DETAILED DESCRIPTION OF EMBODIMENTS

The compositions in Examples 1-7 are specified as follows: *Panax ginseng* is selected as a monarch drug of the compositions. *Panax ginseng* tastes sweet and a little bitter, and is warm and mild-natured. *Panax ginseng* is governed by the spleen, lung and heart meridians. *Panax ginseng* can reinforce vital energy, invigorate spleen for benefiting lung, promote the secretion of saliva and calm the nerves. *Panax ginseng* is selected as a representative drug for invigorating Qi and strengthening the spleen in the composition. Spleen governs movement and transformation, and is the foundation of acquired constitution; the nourishment of Qi and spleen makes body full of energy and blood, and full of vitality. Weak Qi and spleen lead to Weak movement and transformation, causing damp obstruction, Qi stagnation and blood stasis. Spleen prefers dryness to dampness, if the spleen is damp, movement and transformation are abnormal. *Wolfiporia cocos* tastes sweet and slight, is mild-natured. *Wolfiporia cocos* is governed by heart, lung, spleen and kidney channels, capable of clearing damp and promoting diuresis, strengthening spleen and calming heart. *Wolfiporia cocos* serves as the representative drug for strengthening spleen and clearing damp in the composition, and is a ministerial drug of the composition. Nattokinase is a kind of serine protease produced by *Bacillus subtilisl* natto during soybean fermentation, found and named by a Japanese doctor, Hiroyuki Sumi. Nattokinase dissolves thrombus by direct and indirect ways. Nattokinase may stimulate vascular endothelial cells to produce a tissue-type plasminogen activator (t-PA). t-PA activates plasminogen into plasmin to dissolve fibrous proteins, thus dissolving thrombus directly. Meanwhile, prourokinase in human body is activated into urokinase; urokinase and t-PA co-activate plasminogen, thus indirectly dissolving thrombus. Therefore, the thrombolysis activity of nattokinase is about four times of plasmin. Nattokinase is selected as the representative drug for promoting the circulation of blood and removing stasis in the composition, and is another ministerial drug of the prescription. Compound types of gut microbiota substrates are combined in the composition. The substrates basically include different structures of water-soluble dietary fibers mainly derived from plants, including pectin, glucomannan, alginic acid and guar gum. Pectin is a kind of water-soluble dietary fiber which is abundant in fruits; galacturonic acid having carboxyl is connected with methyl galacturonicate whose carboxyl is esterified by methyl in a linear chain. *Pericarpium citri reticulatae* (total pectin is about 33%) rich in multiple types of pectin is selected in the composition (Akiko Kawabata, et al, Journal of Nutrition, 1974). *Pericarpium citri reticulatae* is warm-natured and governed by lung and spleen channels, and has the effects of regulating Qi and strengthening spleen, eliminating dampness to reduce phlegm. Glucomannan is a kind of water-soluble dietary fiber which is abundant in konjak plants. Glucomannan mainly consists of glucose and its diastereoisomers; β (1-4) bonds form a backbone chain, some form branched chains via β (1-3) or β (1-6) bonds. Alginic acid is a kind of water-soluble dietary fiber contained in brown seaweed, and is a linear polymer, divided into two types of β-D-mannuronic acid (M) and C-5 epimer α-L-guluronic acid (G) (both are carboxyl-carrying monosaccharides) (1-4). Crude laminarin is also called laminarin and laminaran, and mainly contains algin, fucoidan, fucose and other components, and formed by linking β(1-3)-glucan to some β(1-6)-glucosidic bonds, and is a neutral glucan. Guar gum is a kind of water-soluble dietary fiber derived from the seeds of annual leguminous plants cultivated in Pakistan and India; guar gum is a kind of polysaccharide composed of repetitive units; the backbone consists of mannose and the side chain consists of galactose. *Psyllium* seed husk contains about 90% dietary fiber, and is a mixture of insoluble and water-soluble dietary fibers. *Psyllium* seed husk contains about 18% heteropolysaccharide xylan, and is a kind of water-soluble dietary fiber. The above compound types of water-soluble dietary fibers may provide respective substrates demanded for different types of gut microbiota. The water-insoluble dietary fiber includes cellulose, hemicellulose, and lignin. *Wolfiporia cocos* is a kind of dry sclerotium of *Poria cocos* (Schw.) Wolf. *Wolfiporia cocos* not only has the effects of strengthening spleen and clearing damp and thus, serves as a ministerial drug of the composition, and but also contains more than 90% water-insoluble dietary fiber and thus, has adjuvant effects. *Psyllium* seed husk contains about 72% water-insoluble dietary fibers. The water-insoluble dietary fiber may provide a small amount of substrates for gut microbiota, what's more, may increase the water content of feces several times to hundreds of times, thus increasing the volume of excrement, beneficial to defecation. The above water-soluble and water-insoluble dietary fibers constitute the adjuvant drug of the composition. *Siraitia grosvenorii* is brought into spleen and large intestine channels, and serves as a conductant drug. The mogmside contained in the *Siraitia grosvenorii* is sweeter 300 times than sucrose, and featured by no energy, high sweetness, pure sweet taste and high safety, and is one of the most excellent natural and healthy sweetening agents. Moreover, *Siraitia grosvenorii* also plays the role of improving the taste of the composition.

### Example 1: composition 1

| Composition | | Compatibility principle | Dose (g) |
|---|---|---|---|
| *Panax ginseng* | | Monarch drug | 0.25 |
| *Wolfiporia cocos* | | Ministerial drug | 1.00 |
| Nattokinase (powder) | | | 1000 Fu |
| Water-soluble and water-insoluble dietary fiber | Guar gum | Adjuvant drug | 0.20 |
| | Alginic acid | | 0.20 |
| | Crude laminarin | | 0.20 |
| | *Pericarpium citri reticulatae* | | 0.20 |
| | Glucomannan | | 0.40 |
| | *Psyllium* seed husk | | 1.00 |
| *Siraitia grosvenorii* | | Conductant drug | 0.70 |

The above components were crushed, sieved and mixed well to prepare a unit amount of the composition 1 in the present invention (namely, the amount of the following "per bag"). The composition may exert two-way regulation on the types and functions of gut microbiota, thus improving and maintaining personalized gut microbiota diversity. Moreover, the composition may keep healthy and have treatment or prevention effects on chronic diseases, in particular to metabolic diseases, immune diseases, mental/neurogenic diseases and other diseases incapable of being treated very well at present. A bag of the composition was brewed and taken once or twice per day with warm water.

### Example 2: composition 2

| Composition | | Compatibility principle | Dose (g) |
|---|---|---|---|
| *Panax ginseng* | | Monarch drug | 0.13 |
| *Wolfiporia cocos* | | Ministerial drug | 0.50 |
| Nattokinase (powder) | | | 500 Fu |
| Water-soluble and water-insoluble dietary fiber | Guar gum | Adjuvant drug | 0.10 |
| | Alginic acid | | 0.10 |
| | Crude laminarin | | 0.10 |
| | *Pericarpium citri reticulatae* | | 0.10 |
| | Glucomannan | | 0.20 |
| | *Psyllium* seed husk | | 0.50 |
| *Siraitia grosvenorii* | | Conductant drug | 0.35 |

The formula of this example may be used as an introducing prescription, or applied to the inadaptable subjects, such as, patients accompanied with continuous abdominal distension, or stomachache after taking the composition 1. The composition may exert two-way regulation on the types and functions of gut microbiota, thus improving and maintaining personalized gut microbiota diversity. Moreover, the composition may keep healthy and have treatment or prevention effects on chronic diseases, in particular to metabolic diseases, immune diseases, mental/neurogenic diseases and other diseases incapable of being treated very well at present. A bag of the composition (namely, the components and contents as shown in the above corresponding table) was brewed and taken once or twice per day with warm water.

### Example 3: composition 3

| Composition | | Compatibility principle | Dose (g) |
|---|---|---|---|
| *Panax ginseng* | | Monarch drug | 0.50 |
| *Wolfiporia cocos* | | Ministerial drug | 2.00 |
| Nattokinase (powder) | | | 2000 Fu |
| Water-soluble and water-insoluble dietary fiber | Guar gum | Adjuvant drug | 0.4 |
| | Alginic acid | | 0.4 |
| | Crude laminarin | | 0.4 |
| | *Pericarpium citri reticulatae* | | 0.4 |
| | Glucomannan | | 0.8 |
| | *Psyllium* seed husk | | 2.00 |
| *Siraitia grosvenorii* | | Conductant drug | 0.70 |

The formula in the example is suitable for the patient whose feces is not improved obviously or improved slowly towards the intermediate type (type 4) of the Bristol Stool Scale after taking the composition 1 in Example 1. The above components were crushed, sieved and mixed well to prepare a unit amount of the composition 3 in the present invention. The composition may exert two-way regulation on the types and functions of gut microbiota, thus improving and maintaining personalized gut microbiota diversity. Moreover, the composition may keep healthy and have treatment or prevention effects on chronic diseases in particular to metabolic diseases, immune diseases, mental/neurogenic diseases and other diseases incapable of being treated very well at present. A bag of the composition was brewed and taken once to twice per day with warm water.

### Example 4: composition 4

In this example, the total extract of *Panax ginseng* may be directly obtained from the extract of *Panax ginseng* purchased on the market, or the primary extract product of *Panax ginseng.*

| Composition | | Compatibility principle | Dose (g) |
|---|---|---|---|
| Total extract of *Panax ginseng* | | Monarch drug | 0.01 |
| *Wolfiporia cocos* | | Ministerial drug | 1.00 |
| Nattokinase (powder) | | | 1000 Fu |
| Water-soluble and water-insoluble dietary fiber | Guar gum | Adjuvant drug | 0.20 |
| | Alginic acid | | 0.20 |
| | Crude laminarin | | 0.20 |
| | *Pericarpium citri reticulatae* | | 0.20 |
| | Glucomannan | | 0.40 |
| | *Psyllium* seed husk | | 1.00 |
| *Siraitia grosvenorii* | | Conductant drug | 0.70 |

The above components were crushed, sieved and mixed well to prepare a unit amount of the composition 4 in the present invention. The composition 4 may replace the composition 1. The composition may exert two-way regulation on the types and functions of gut microbiota, thus improving and maintaining personalized gut microbiota diversity. Moreover, the composition may keep healthy and have treatment or prevention effects on chronic diseases in particular to metabolic diseases, immune diseases, mental/neurogenic diseases and other diseases incapable of being treated very well at present. A bag of the composition was brewed and taken once or twice per day with warm water.

### Example 5: composition 5

| Composition | Compatibility | Dose | |
|---|---|---|---|
| | | principle | (g) |
| Total extract of *Panax ginseng* | | Monarch drug | 0.005 |
| *Wolfiporia cocos* | | Ministerial drug | 0.50 |
| Nattokinase (powder) | | | 500 Fu |
| Water-soluble and water-insoluble dietary fiber | Guar gum | Adjuvant drug | 0.1 |
| | Alginic acid | | 0.1 |
| | Crude laminarin | | 0.1 |
| | *Pericarpium citri reticulatae* | | 0.1 |
| | Glucomannan | | 0.2 |
| | *Psyllium* seed husk | | 0.5 |
| *Siraitia grosvenorii* | | Conductant drug | 0.35 |

The formula of this example may be used as an introducing prescription, or applied to the inadaptable patients, such as, patients accompanied with continuous abdominal distension, or stomachache after taking the composition 1 provided in Example 1 or the composition 4 provided in Example 4. The composition 5 may replace the composition 2 provided in Example 2. The composition may exert two-way regulation on the types and functions of gut microbiota, thus improving and maintaining personalized gut microbiota diversity. Moreover, the composition may keep healthy and have treatment or prevention effects on chronic diseases in particular to metabolic diseases, immune diseases, mental/neurogenic diseases and other diseases incapable of being treated very well at present. A bag of the composition was brewed and taken once or twice per day with warm water.

### Example 6: composition 6

| Composition | Compatibility principle | Dose (g) | |
|---|---|---|---|
| Total extract of *Panax ginseng* | Monarch drug | 0.02 | |
| *Wolfiporia cocos* | Ministerial drug | 2.00 | |
| Nattokinase (powder) | | 2000 Fu | |
| Water-soluble and water-insoluble dietary fiber | Guar gum | Adjuvant drug | 0.4 |
| | Alginic acid | | 0.4 |
| | Crude laminarin | | 0.4 |
| | *Pericarpium citri reticulatae* | | 0.4 |
| | Glucomannan | | 0.8 |
| | *Psyllium* seed husk | | 2 |
| *Siraitia grosvenorii* | | Conductant drug | 0.70 |

The formula in the example is suitable for the patient whose feces is not improved obviously or improved slowly towards the intermediate type (type 4) of the Bristol Stool Scale after taking the composition 4 provided in Example 4. The composition 6 may replace the composition 3 provided in Example 3. The above components were crushed, sieved and mixed well to prepare a unit amount of the composition 6 in the present invention. The composition may exert two-way regulation on the types and functions of gut microbiota, thus improving and maintaining personalized gut microbiota diversity. Moreover, the composition may keep healthy and have treatment or prevention effects on chronic diseases in particular to metabolic diseases, immune diseases, mental/neurogenic diseases and other diseases incapable of being treated very well at present. A bag of the composition was brewed and taken once to twice per day with warm water.

### Example 7: composition 7

The composition was specified below: almond (0.25-1 g) and perilla seed (0.25-1 g) were added to the composition as an adjuvant drug. The composition 7 is suitable for the patient suffering gut microbiota imbalance and diagnosed with constipation due to intestinal dryness based on traditional Chinese medicine (TCM). The composition 7 may be used flexibly according to TCM diagnosis. The almond dietary fiber has a content greater than 10% and the abundant unsaturated fatty acids therein has the effect of lubricating the intestines. Perilla seed is rich in unsaturated fatty acids (>50%) and abundant essential amino acids. That is, perilla seed may provide substrates for gut microbiota, and also have the effect of lubricating the intestines and relaxing the bowels for constipation due to intestinal dryness.

The above components were crushed, sieved and mixed well to prepare a unit amount of the composition 7 in the present invention. The composition 7 is suitable for constipation patients. The composition may exert two-way regulation on the types and functions of gut microbiota, thus improving and maintaining personalized gut microbiota diversity. Moreover, the composition may keep healthy and have treatment or prevention effects on chronic diseases in particular to metabolic diseases, immune diseases, mental/neurogenic diseases and other diseases incapable of being treated very well at present. A bag of the composition was brewed and taken once or twice per day with warm water.

Ingredients for strengthening spleen and invigorating Qi include but not limited to a crude drug or extract of *Panax ginseng* and/or *Panax quiquefolium.*

The ingredient for clearing damp includes but not limited to a crude drug or extract of *Wolfiporia cocos.*

The ingredient for promoting the circulation of blood includes but not limited to a crude drug or extract of nattokinase or natto.

The water-soluble dietary fiber includes but not limited to one or more of glucomannan, pectin, alginic acid, β-glucan and inulin. Food ingredients or medicinal materials, or the like capable of extracting the above these components are certainly available.

The water-insoluble dietary fiber includes but not limited to one or more of cellulose, hemicellulose, and lignin.

In the daily dose (weight) of the composition, the water-soluble dietary fiber and the water-insoluble dietary fiber are calculated by total weight percentage of the composition, including 0.01-2 g crude drug of *Panax ginseng* and *Panax quiquefolium,* or 0.001-0.2 g extract of *Panax ginseng* and *Panax quiquefolium,* 0.1-5 g crude drug of *Wolfiporia cocos,* or 0.001-2 g extract of *Wolfiporia cocos,* 10-50 g natto powder, or 500-4000 Fu nattokinase; 0.1-3 g crude drug of *Siraitia grosvenorii,* or 0.001-0.2 g extract of *Siraitia grosvenorii.* The water-soluble dietary fiber accounts for 10%-70% by the total dose of the formula; the water-insoluble dietary fiber accounts for 10%-70% by the total dose of the formula.

The present invention is achieved by the following technical solutions: the technical solutions of the present invention are proved from four levels of molecule (gene analysis of intestinal bacteria), function (functional genomes analysis), system phenotype (Bristol Stool Scale), and some representative laboratory detection indexes, including blood lipid (total cholesterol, triglyceride, low density cholesterol and high density cholesterol), blood glucose (fasting blood-glucose, glucose tolerance test), serum uric acid, renal function indexes (serum creatinine and estimated glomerular filtration rate), and liver function indexes (alanine aminotransferase and serum aspartate aminotransferase). The composition may improve and maintain the personalized gut microbiota diversity via two-way regulation on gut microbiota, thus preventing or treating various diseases associated with gut microbiota imbalance.

The molecular level analysis was performed by OTU clustering and species annotation. Vsearchv2.4.4 was used for (OTU) analysis; the sequence was subjected to repetitive sequence removal (--derep_fulllength), clustering (--cluster_fast,-id 0.97) and chimera removal (--uchime_ref) (Rognes2016), then clustered into OTUs according to 97% similarity by default. Default parameters were used to select an OTU representative sequence; and the representative sequence was subjected to species annotation via VSEARCH based on SILVA128 database (Quastet al. 2013) to generate an OTU list, thus obtaining the community composition in each sample at the level of Phylum, Class, Order, Family, Genus and Species, as well as the abundance and classification of all the OTUs in each sample. For the quality control of the raw sequence data, the data size of the tags and clean tags was firstly judged whether of satisfying the sequencing requirements; generally, when the data size of the tags was up to 30,000 reads above, the sequencing requirements were satisfied. Reads of the mean clean tags of the 16 s sample in this test were 107670.08, completely capable of satisfying the sequencing requirements. OTUs whose content was lower than 0.001% of the total sequences in the sample were not taken in this experiment due to the current detection sensitivity.

For the functional genome analysis, the number of Species and Genus of the raw 16s sequencing data was standardized. A comparatively mature tool PICRUSt (PICRUSt Nature Biotechnology, 1-10. 8 2013) which is developed at the earliest and used for predicting microflora functions based on 16S rRNA gene sequence was then utilized to obtain the predictive functions of the genome.

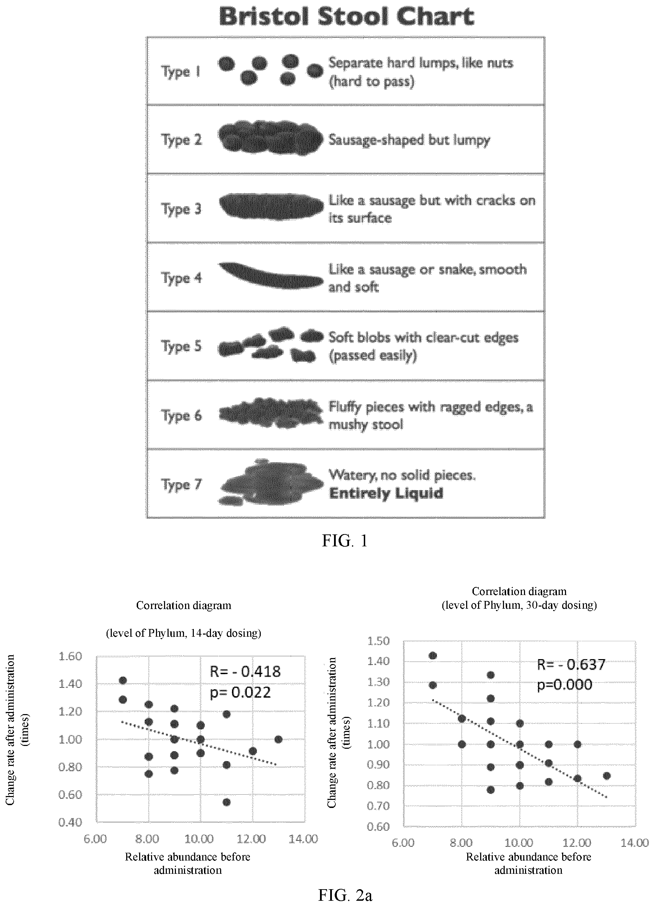

As for the system phenotype analysis, Bristol Stool Chart, as shown in FIG. 1, was utilized as an integrated index of gut microbiota diversity for analysis to obtain Bristol Stool Score. The judgment method is as follows: the point of type 4 (normal faeces) is 1, the point of types 3 and 5 is 2, the point of types 2 and 6 is 4, the point of types 1 and 7 is 6. The higher the point is, the more abnormal the faeces is. Bristol Stool Scale is the best detection index to reflect the normality of the gut microbiota microecosystem in the level of body phenotype (Falony et al. Science 2016).The objective of the present invention is to prove that the composition may reconstruct the structure of gut microbiota via two-way regulation, thus promoting the Bristol Stool Score to transform towards the intermediate phenotype (type 4).That is, constipation may be eliminated and diarrhea may be improved instead of simply improving constipation or diarrhea.

Modified Cleveland Stool Score, as an auxiliary index of defecation, is also taken in the present invention. The method may be used to achieve better comprehensive analysis on the improvement effects on constipation based on the point of Bristol Stool Scale plus indexes listed in the two tables.

| | Point 0 | Point 1 | Point 2 | Point 3 | Point 4 |
|---|---|---|---|---|---|
| Defecation times | More than 3 times/week | Twice/ week | Once/w eek | Once below/week | Once below/month |
| Difficult defecation | No | Acciden tal^{∗} | Someti mes^{∗} | Often^{∗} | Always^{∗} |
| Feeling of residual feces | No | Acciden tal | Someti mes | Often | Always |
| Stomachache | No | Acciden tal | Someti mes | Often | Always |
| Defecation time | 5 min below | 5-9 min | 10-19 min | 20-29 min | 30 min above |
| Auxiliary defecation | No | Laxativ e | Enema | | |
| Desire to defecate, but no defecation Times/24 h | 0 | 1-3 | 4-6 | 7-9 | More than 10 times |
| History of constipation (year) | 0 | 1-5 | 6-10 | 11-20 | More than 21 years |
| ^{∗}Accidental: once below/month; sometimes: once/more than one month-once below/week; | | | | | |
| often: once/more than one week-once below/day; always: more than once/day | | | | | |

The administration method, administration time, detection method and statistical treatment are concluded and specified as follows:

| Measurem ent index | Sample size (Male/Fem ale) | Age | Medicame nt taken ^{∗} | Administra tion time (d) | Detection/ Measurem ent method | Analysis method | Statistical method |
|---|---|---|---|---|---|---|---|
| 16s RNA sequencing analysis of microorga nism | 30 (20/10) | 26-74 | Compositi ons 1-7 were regulated based on TCM diagnosis and administra tion reaction | 14 | 16s RNA sequencing analysis of microorga nism | Gut microbio ta types were classifie d accordin g to the analysis on the Operatio nal Taxono mic Units | Pearson correlation coefficient was taken to the correlation, and non-correla tion was excluded by paired T test; paired T test was used for intergroup control |
| | 30 (20/10) | 26-74 | | 30 | | PICRUS t analysis of 16S rRNA gene sequence | |
| Bristol Stool Scale | 28 (19/9) | 26-74 | | 14 30 | Self-observe and report | Analysis was performe d accordin g to the followin g types of points: Type 4=1; types 1,7=6; types 2,6=4; types 3,5=2 | Paired T test was used for the difference before and after administrat ion of intergroup control |
| | 24 (17/7) | 26-74 | | | | | |
| Modified Cleveland Stool Scale | 28 (19/9) | 26-74 | | 14 | Self-obser ve and report | Quantitat ive analysis was performe d on the basis of Bristol Stool Scale and other defecation symptom s (the specific method is referring to the descripti on in this text) | Paired T test was used for the difference before and after administrat ion |
| | 24 (17/7) | 26-74 | | 30 | | | |

| | | | | | | | |
|---|---|---|---|---|---|---|---|
| ^{∗}Administration method and time are specified as follows: 1) the composition 1 or 4 was taken first (in this example, the subsequent test data was obtained on the condition that the composition 1 was mixed with 250-300 ml warm water and taken twice per day as the starting formula). After taking the composition, some of patients would suffer abdominal distension and even stomachache, and the symptoms of abdominal distension and stomachache would be improved or removed within two or three days generally, and then, the composition may be continuously taken. 2) If the reaction of abdominal distension and even stomachache last a week around, the composition 2 or 5 was used for replacement (in this example, the subsequent test data was obtained on the condition that the composition 2 was taken as the adjustment formula). After the patient adapted to the composition one week later, if the condition of the feces (Bristol Stool Scale) was good, the composition 2 or 5 may serve as the maintenance prescription. If the condition of feces (Bristol Stool Scale) had no obvious response, the composition 1 or 4 was taken again once the abdominal distension or stomachache was improved or removed. 3) If the condition of feces (Bristol Stool Scale) had no obvious response with one week dosing of the composition 1 or 4, the composition 3 or 6 was used (in this example, the subsequent test data was obtained on the condition that the composition 3 was taken as the adjustment formula). If the condition of feces (Bristol Stool Scale) had good response, the composition 1 or 4 was taken again as the maintenance prescription. 4) The patients accompanied with constipation started to take the composition 7. When the constipation was improved after administration, the composition 1 or 4 was taken as the maintenance prescription. | | | | | | | |

Moreover, it should be noted that in the table above, the subjects administered for 14 days and the subjects administered for 30 days were from the same group of patients. Feces of these subjects were tested twice to confirm the intervention effect of the composition on the subacute (14 days) and long-term (30 days) of gut microbiota. Meanwhile, repeatability of the drug effect was also verified by the comparison of the two test results.

The composition has good two-way regulation effect on Phylum, Class, Order, and Family. The so-called two-way regulation effect mainly refers that the composition may selectively enhance the relatively less abundant strain and their function. The two-way regulation effect is reflected in that the lower the relative abundance of the bacteria, or functional genomes is before administration, the higher the relative abundance of the bacteria, or functional genomes is after administration. The increase of the relatively less abundant intestinal bacteria or functional genomes proportionally lower the ratio of dominant bacteria or functional genomes, capable of strengthening and maintaining the microecological balance of gut microbiota. It will be abbreviated for two-way regulation in the following description of the present invention, and will be not repeated any more.

FIGS. 2a-2d respectively show correlation diagrams of relative abundance of bacteria before and after administration at the level of Phylum, Class, Order, and Family; (X-coordinate is the relative abundance before administration; Y-coordinate is the change rate of the abundance after administration, namely, relative abundance after administration/relative abundance before administration, for example, "1" indicates no change of the relative abundance after administration; ">1" indicates the increase of the relative abundance after administration; "<1" indicates the decrease of the relative abundance after administration; the dark spot represents the mean value of each bacterium at the level of Phylum, Class, Order, and Family). The left figure shows a correlation diagram with 14-day dosing. The right picture shows a correlation diagram with 30-day dosing.

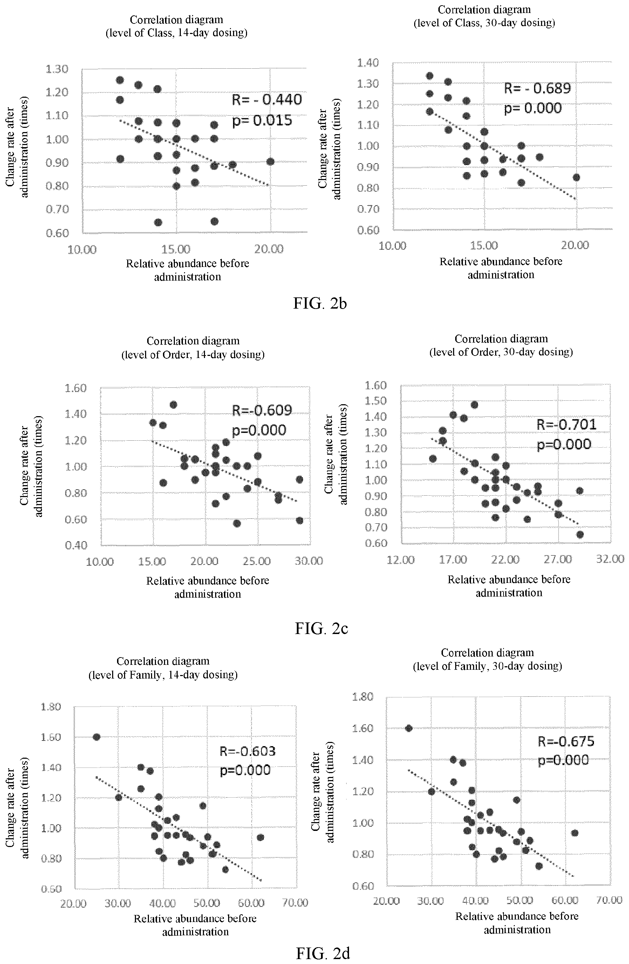

As shown in FIG. 2a, at the level of Phylum, the lower abundance the bacterium is, the higher increment is observed after administration. with 14-day dosing, it has appeared an obvious negative correlation (the left figure of FIG. 2a), and there are remarkable statistical significances; with 30-day dosing (the right picture of FIG. 2a), the significant statistic differences are further increased. At the levels of Class (FIG. 2b), Order (FIG. 2c), and Family (FIG. 2d), there are obvious negative correlations with both 14-day (corresponding to the left figure) and 30-day (corresponding to the right picture) later after administration; and there are remarkable statistical significances. The above results indicate that the composition has good two-way regulation at the levels of Phylum, Class, Order and Family, and may inhibit the relative abundance of dominant bacteria and enhance the less abundant bacteria, thereby improving the functional balance and diversity of gut microbiota.

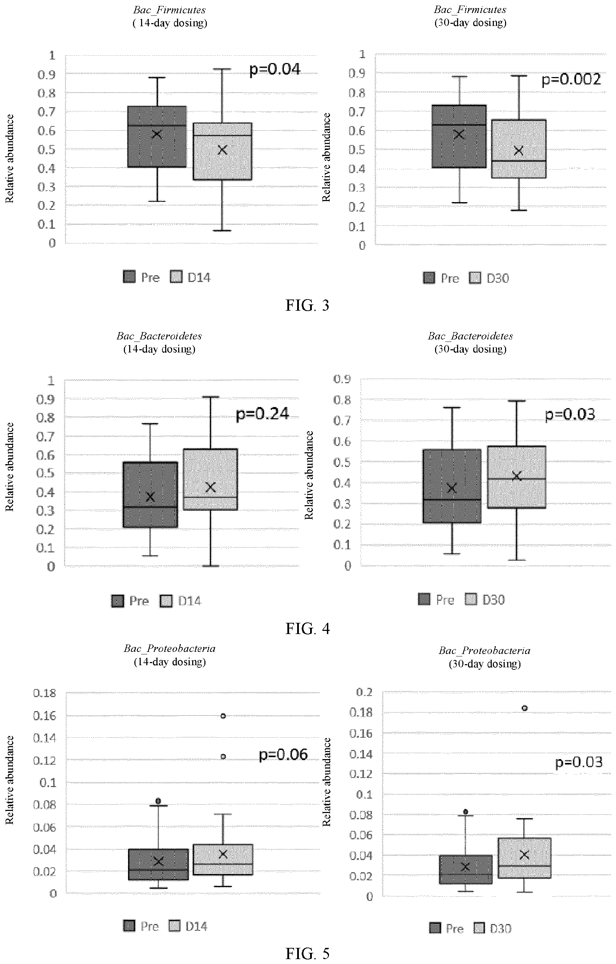

The composition has an inhibitory effect on the most dominant bacterium at the level of Phylum, *Bac _Firmicutes.* As shown in FIG. 3 (Y-coordinate is relative abundance; the transverse line in the box plot represents a median; X represents a mean value; straight lines at both ends represent a scope of 75%. Coordinates or charts in the subsequent FIG. 4, FIG. 5, FIG. 8, FIG. 9a, FIG. 9b, FIG. 9c, FIG. 12, FIG. 13b, FIG. 13c, FIG. 13d, FIG. 16, FIG. 17, FIG. 18, FIG. 20a, FIG. 20b, FIG. 21b, FIG. 24, FIG. 26b, FIG. 27, FIG. 29, FIG. 31, FIG. 33b, FIG. 34a, FIG. 34b, FIG. 35, and FIG. 36 have the meanings the same as those in FIG. 3). With 14-day dosing, the relative abundance of *Bac _Firmicutes* decreases 14.5%, and there are remarkable statistical significances. with 30-day dosing, the relative abundance of *Bac Firmicutes* continuously decreases 15% above, and statistical significances are greater than that of the 14-day administration. The above result shows the effectiveness and long-term intervention effect of the composition. Obesity patients have increased *Bac Firmicutes,* and the most effective bariatric surgery can decrease the abundance of *Bac Firmicutes* (Muscogiuri et al. 2019; Ejtahed et al. 2018). It indicates that the composition may improve the body weight of patients, the frequently accompanied metabolic syndromes, type 2 diabetes (type II diabetes), fatty liver and the like by reducing the relative abundance of *Bac Firmicutes* without highly-invasive surgery. The current researches believe that *Bac _Firmicutes* can ferment the dietary fiber incapable of being digested by human, thus providing extract energy sources for human being. This is one of the important contributions to help people get through famine in the hungry age. However, in the satiation/overfeeding age, such an action causes the side effect of overnutrition.

The composition significantly improves the abundance of the second dominant bacterium at the level of Phylum, *Bac Bacteroidetes.* As shown in FIG. 4, the relative abundance of *Bac Bacteroidetes* increases 13.8% with 14-day dosing. With 30-day dosing, the relative abundance increases to 15%, and there are remarkable statistical significances. The above result proves the effectiveness and long-term intervention effect of the composition. The relative abundance of *Bac Bacteroidetes* decreases in obesity patients. The composition is expected to improve the body weight of patients, as well as frequently accompanied metabolic syndromes, or type 2 diabetes, fatty liver and the like by improving the relative abundance of *Bac Bacteroidetes* (Muscogiuri et al. 2019). Moreover, *Bac Bacteroidetes* in the patients with inflammatory bowel disease or Crohn's disease decreases significantly, which is one of the reasons to cause the imbalance of gut microbiota microecosystem (Presti at al. 2019). The composition may improve the abundance of *Bac _Bacteroidetes,* and used for a medicament or a health-care product or food, and may be expected to treat and prevent the chronic inflammatory bowel diseases caused by immunologic dysfunction.

The composition significantly improves the abundance of the third dominant bacterium at the level of Phylum, *Bac_Proteobacteria.* As shown in FIG. 5, the relative abundance of *Bac Bacteroidetes* increases 23.9% with 14-day dosing. With 30-day dosing, the relative abundance increases 41% above, and there are remarkable statistical significances. The above result proves the effectiveness and long-term intervention effect of the composition.

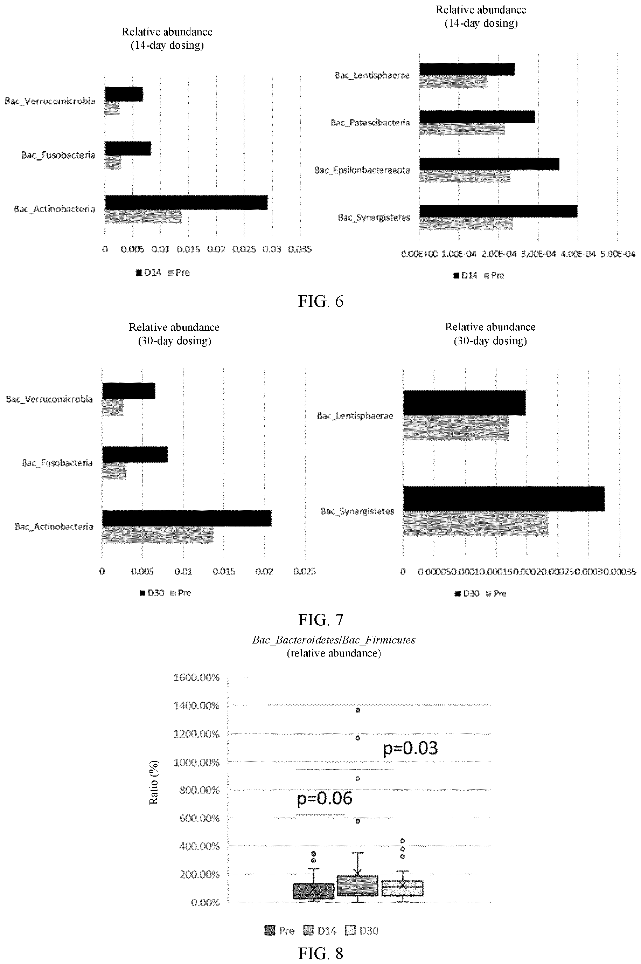

The composition may improve the abundance of other less abundant bacteria at the level of Phylum. As shown in FIG. 6 (X-coordinate is relative abundance). For most of the less abundant bacteria at the level of Phylum above the detection limit, the relative abundance is improved with 14-day dosing, including 214.15% *Bac_Actinobacteria,* 276.83% *Bac_Fusobacteria,* 264.62% *Bac_Verrucomicrobia,* 169.6% *Bac_Synergistetes,* 154.25% *Bac_Epsilonbacteraeota,* 135.36% *Bac_Patescibacteria,* and 140.67% *Bac_Lentisphaerae.* In the above less abundant bacteria at the level of Phylum, the relative abundance of *Bac_Actinobacteria* and *Bac_Fusobacteria* may help enhancing immunity or increasing the therapeutic effects of anti-cancer drugs (Helmink et al. Nature Medicine 2019). The composition may enhance immunity or increase the perceptibility of the host human body to anti-cancer drugs by enhancing these less abundant bacteria at the level of Phylum.

As shown in FIG. 7 (X-coordinate is relative abundance). with 30-day dosing, the same trend is kept: 152.14% *Bac_Actinobacteria,* 271.65% *Bac_Fusobacteria,* 251.06% *Bac_Verrucomicrobia,* 131.05% *Bac_Tenericutes* and 138.46% *Bac_Synergistetes.* The above results prove the effectiveness and long-term intervention effect of the composition on the less abundant bacteria at the level of Phylum.

To sum up, the composition may inhibit the relative abundance of dominant bacteria and enhance the relative abundance of the less abundant bacteria (or relatively less abundant dominant bacteria) at the level of Phylum, and shows good two-way regulation. The enhancement of the less abundant bacteria at the level of Phylum indicates that the composition may help the reproduction and maintenance of low-abundance bacteria, thus helping improving and maintaining the diversity of gut microbiota, and keeping the health condition of the gut microbiota microecosystem.

As shown in FIG. 8 (Y-coordinate represents a *Bac_Bacteroidetes*/*Bac_Firmicutes* ratio), the composition improves the *Bac_Bacteroidetes*/*Bac_Firmicutes* ratio. With 14-day dosing, the composition increases the median of the *Bac_Bacteroidetes*/*Bac_Firmicutes* ratio by 5%. With 30-day dosing, the median even increases to 10%, and there are remarkable statistical significances. The above result proves the effectiveness and long-term intervention effect of the composition. The increase of the *Bac_Bacteroidetes*/*Bac_Firmicutes* ratio may improve the metabolic functions of the host, and particularly have useful effects on obesity and diabetes, which have been proved by multiple researches and clinical observations (Qin J. at al., Nature 2010; Karlsson FH, Nature 2013).The composition may improve the metabolic diseases by adjusting the *Bac_Bacteroidetes*/*Bac_Firmicutes* ratio.

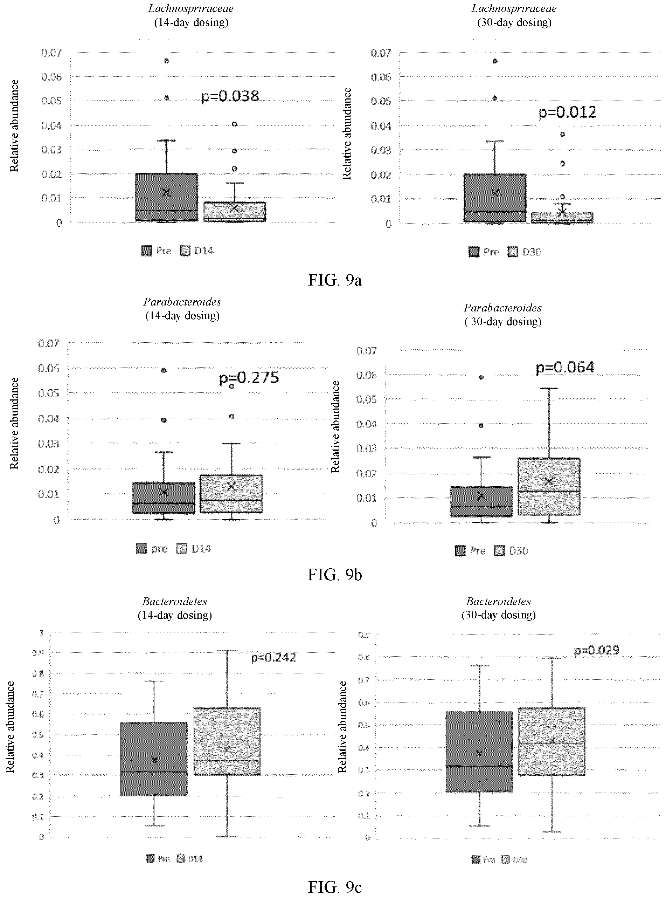

Gut-brain axis is an existing concept. The research progress on gut microbiota brings new contents for such a concept. For example, autism is always accompanied with digestive tract symptoms such as, constipation and partial eclipse and thus, has been concerned extensively in the correlation with gut microbiota for the past few years. The latest shocking discovery has proved that the decrease of several specific bacterial genus, including *Bacteroides* and *Parabacteroides,* and the increase of *Lachnospriraceae* are the reasons (causative) to cause abnormal behaviors of autism (Sharon et al. Cell 2019). The composition may significantly decrease the relative abundance of *Lachnospriraceae* (FIG. 9a), and simultaneously increase the relative abundance of *Parabacteroides* (FIG. 9b) and *Bacteroides* (FIG. 9c). It indicates that the composition is expected to improve the symptoms of autism from the cause of disease. The composition is used for a medicament or a health-care product or food, and may have treatment and prevention effects on autism and other diseases caused by gut-brain axis dysfunction.

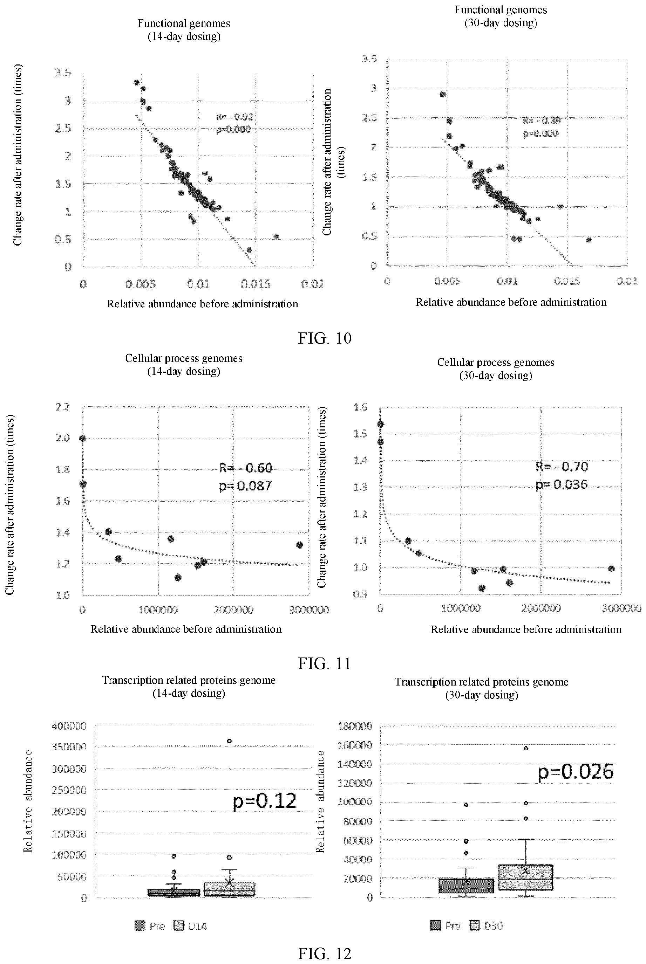

Researches show that compare to the composition of bacteria, there is always a smaller inter-individual functional variation, which may reflect the condition of the gut microbiota microecosystem better. The present invention further specifies the effects of the composition from the functional level by analyzing the functional genomes of gut microbiota. FIG. 10 (X-coordinate is the relative abundance before administration, and Y-coordinate the change rate of the relative abundance after administration; the filled dots in the figure show mean values of metabolic pathways) shows the related analysis results of the changes of the general functional metabolic pathways (213 pathways in total) after administration (the left figure shows the result with 14-day dosing, and the right picture shows the result with 30-day dosing) and the relative abundance before administration; the dotted line in the figure represents a linear trend. As shown in the figure, the composition of the present invention shows excellent two-way regulation effects; the correlation coefficient is -0.9 around, and there is a highly significant negative correlation (p=0.000). Such a result proves that the composition has two-way regulation on the overall metabolic pathways, thus showing its usefulness in the aspects of improving, strengthening and maintaining the functional balance and diversity of gut microbiota from the overall level.

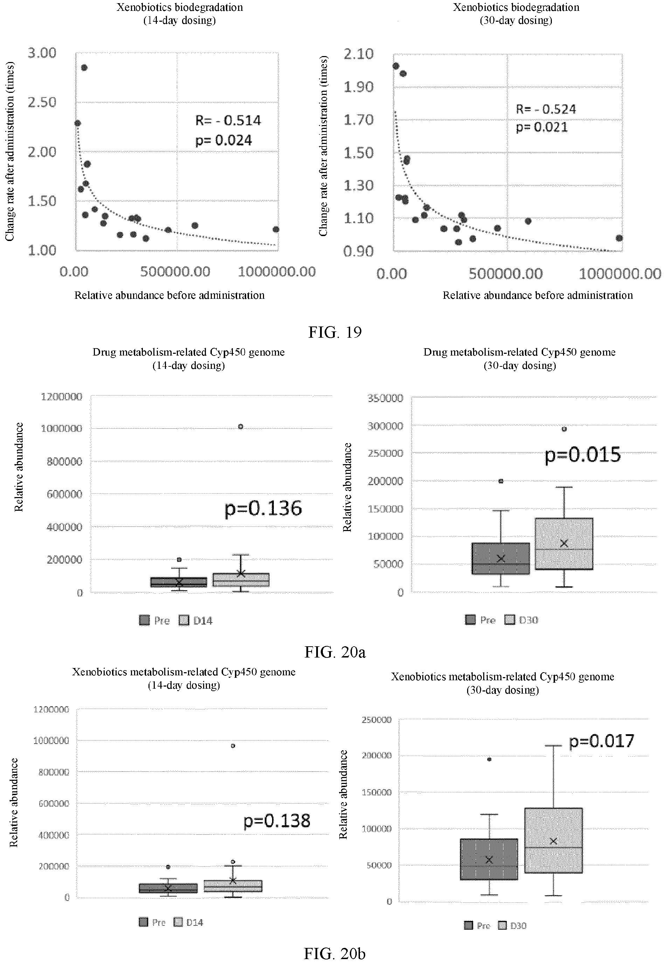

The present invention further performs in-depth studies on the usefulness of the composition from the following four aspects. Firstly, from the angle of core function of gut microbiota, namely, maintaining self-survival and reproduction by bacteria, FIGS. 11-18 indicate the acceleration of the composition on self-viability of gut microbiota; secondly, FIGS. 19-20 indicate that the composition may enhance the catabolism of gut microbiota to pollen hazardous substances, thus assisting or promoting the health of the host human body; thirdly, FIGS. 21-33b indicate that the composition may promote the gut microbiota to increase the metabolites beneficial to the host human body; finally, FIGS. 34a-38 indicate that the composition may adjust the metabolic genomes of gut microbiota, thus promoting the immune metabolism functions.

As shown in FIG. 11 (X-coordinate is the relative abundance before administration, and Y-coordinate is the change rate of the relative abundance after administration; the filled dots in the figure show mean values of the relative abundance of related genome; the dotted line represents a linear trend), the cellular process summarizes the basic survival functions of gut microbiota, including genetic transcription, cell signal transduction, function and metabolism of mitochondria, and the like. There is certain negative correlation with 14-day dosing (left figure), and the correlation is more obvious with 30-day dosing (right picture), and there are remarkable statistical significances. The dotted line in the figure represents an exponential trend. The composition has a very strong acceleration effect on the least abundant compare to pre-administration regardless of 14- or 30-day dosing. It indicates that the composition has two-way regulation on the cellular process.

FIG. 12 (Y-coordinate represents the relative abundance) indicates that the composition has acceleration on the cell nucleus gene transcription, a subordinate concept of the cellular process. with 14-day dosing (left figure), there is an accelerating trend to the relative abundance of the transcription related proteins gene group; and with 30-day dosing, such kind of acceleration is up to remarkable statistical significance, thus indicating the effectiveness of the composition, in particular to long-term administration.

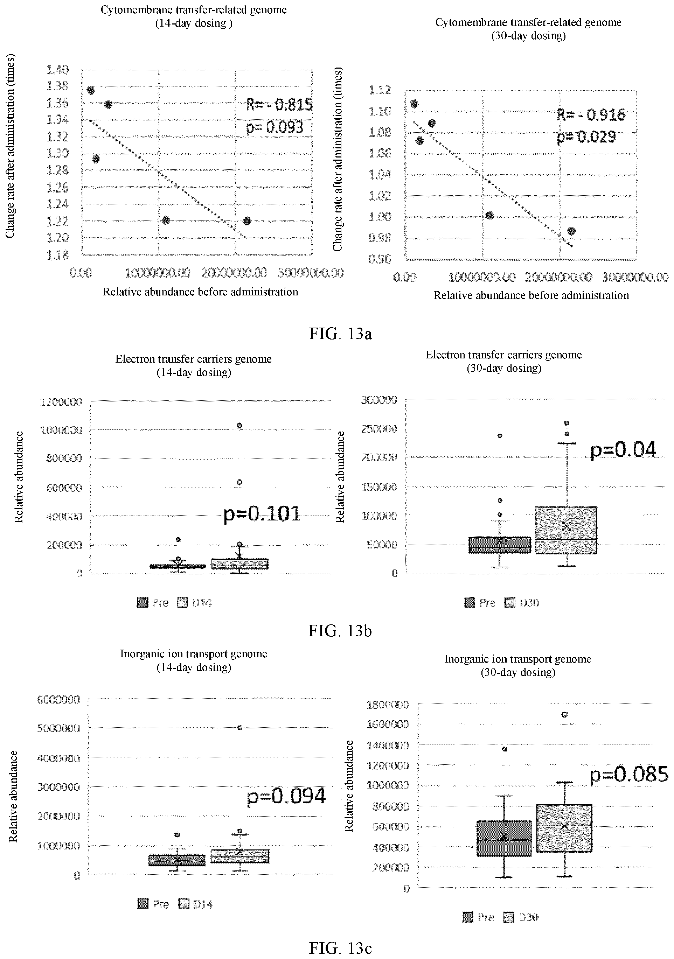

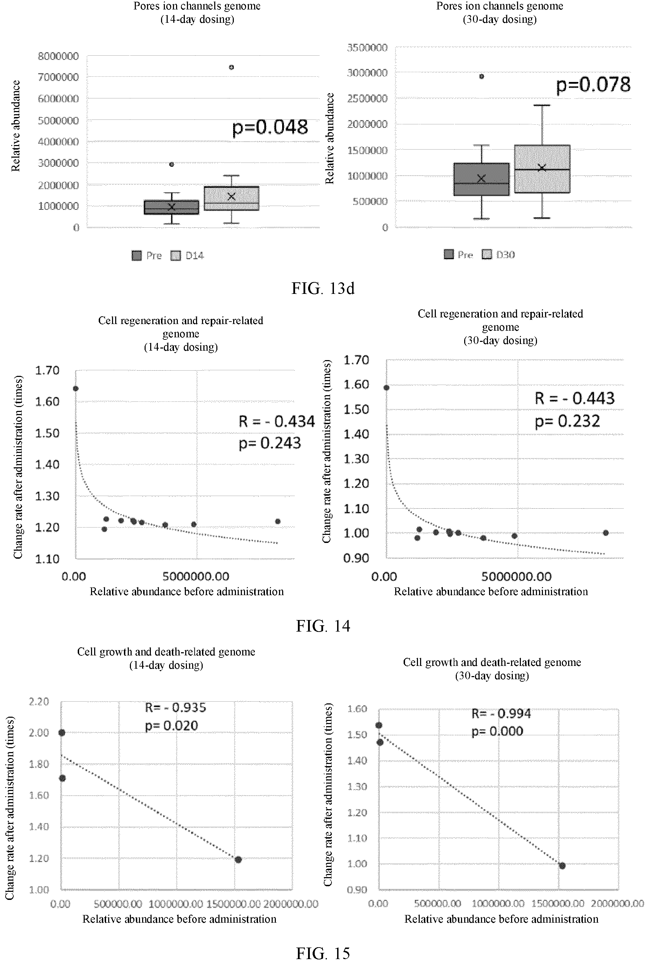

FIGS. 13a-13d (Y-coordinate of FIG. 13a represents the change rate of the relative abundance before and after administration; X-coordinate represents the relative abundance before administration; the dotted line represents a linear trend. Y-coordinates of FIG. 13b-13d represent the relative abundance) indicate the acceleration of the composition on the cytomembrane information transport function, a subordinate concept of the cellular process. The composition has a very strong enhancement effect on the least abundant cytomembrane information transport function genes (Membrane Transport Gene Group) by both 14- and 30-day dosing. It indicates that the composition has two-way regulation on the cytomembrane information transport function. Relevant analysis indicates that the composition has two-way regulation on the cytomembrane information transport function, which is mainly reflected in that the relatively less abundant electron transfer carriers genome, inorganic ion transport genome and pores ion channels genome are enhanced very well. A trend and a statistical significance were observed in groups with 14- and 30-day dosing, respectively. Such an effect may strengthen the cell information transport of less abundant bacteria or flora to enhance their survival and reproduction capacity.

The cellular process includes repair and replication functions of DNA. As shown in FIG. 14 (X-coordinate is the relative abundance before administration, and Y-coordinate is the change rate of the relative abundance after administration; the filled dots in the figure show mean values of the relative abundance of related genome; the dotted line represents an exponential trend). Even though the statistical correlation analysis only shows a weak negative correlation, it can be seen from the figure that the relative abundance of the non-homologous end-joining (with the least abundance) increases by 60% after 14 and 30 days administration. The result indicates the effectiveness of the composition on the less abundant functional genome. Non-homologous end-joining is a kind of special DNA double-strand break repair mechanism that eukaryotic cells link two DNA broken ends with each other independent of DNA homology. The results show that the composition may accelerate eucaryon, also may including intestinal bacteria to use the non-homologous end-joining repair mechanism for DNA repairing and regeneration.

As shown in FIG. 15 (X-coordinate is the relative abundance before administration, and Y-coordinate is the change rate of the relative abundance after administration; the filled dots in the figure show mean values of the relative abundance of related genome; the dotted line represents a linear trend.), the composition also shows a good negative correlation to the life-maintaining functional genome (cell growth and death-related genome) of gut microbiota, and has two-way regulation effects.

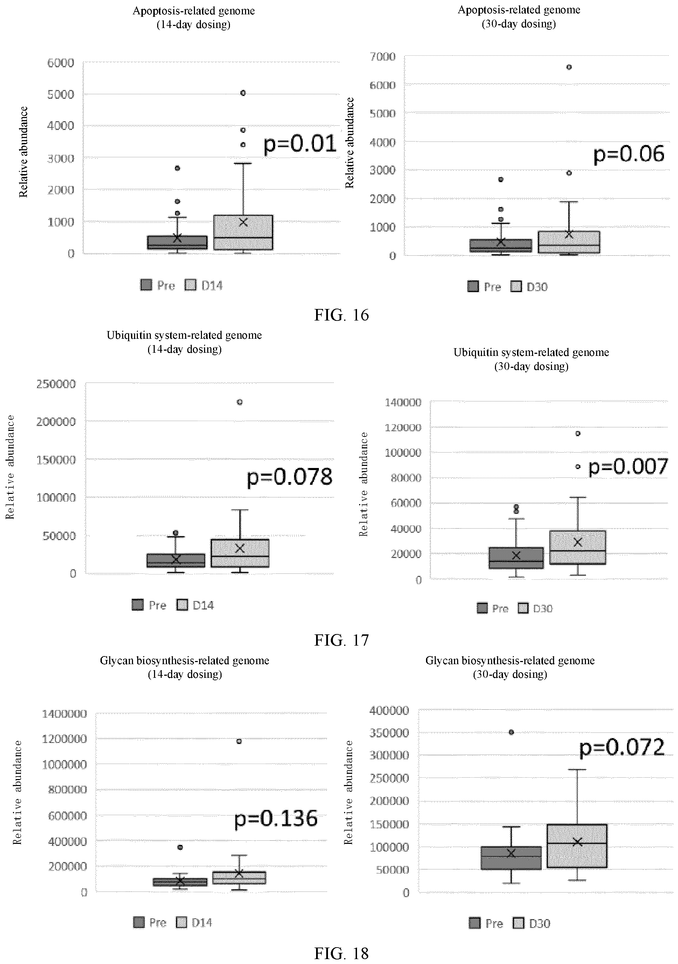

The cellular process includes cell apoptosis and protein degradation through Ubiquitin system. As shown in FIG. 16 (Y-coordinate represents relative abundance), the composition of the present invention may accelerate the process of cell apoptosis. Apoptosis is a process of programmed cell death to maintain the stability of internal environment. Impairment of apoptosis will cause lots of undesirable consequences, the typical one is abnormal cell proliferation, for example, cancer. The acceleration of the composition on cell apoptosis may maintain the normal turnover of cells.

Ubiquitin system is another cell protein degradation chain reaction. Ubiquitin system participates in most of the short-period protein degradation, and participates in lots of cell life processes by regulating the metabolism of intracellular proteins. FIG. 17 (Y-coordinate shows the relative abundance) indicates that the composition may improve the ubiquitin system-related genome with 14-day dosing. It indicates that the composition of the present invention has a functional acceleration trend to the ubiquitin system, and with 30-day dosing, the acceleration effect is further enhanced and has remarkable statistic differences compared with that before administration. The above result indicates a long-term intervention effect of the composition.

Peptidoglycan is an important component of bacterial cell wall. The composition shows certain two-way regulation on Glycan Biosynthesis. As shown in FIG. 18 (Y-coordinate shows the relative abundance), with 14- and 30-day dosing, the relative abundance of glycan biosynthesis-related genome is increased; after taking the composition of the present invention, the glycan biosynthesis shows an increase trend.

Xenobiotics biodegradation is an important function to gut microbiota. Xenobiotics include medicaments, pesticides, plastics or rubber residues, environmental pollutants, food additives, and the like; most of them are harmful to human health. As shown in FIG. 19 (X-coordinate is the relative abundance before administration, and Y-coordinate is the change rate of the relative abundance after administration; the filled dots in the figure show mean values of the relative abundance of related genome; the dotted line represents an exponential trend), the composition may bidirectionally adjust the biodegradation of gut microbiota to xenobiotics. Statistics show that there are significant negative correlations with 14- or 30-day dosing. The fine dotted line shows the acceleration of the composition on less abundant functional genome. Such kind of selective acceleration is of significance to human health.

The table below summarizes the acceleration effects of the composition on some important functional genomes of xenobiotics biodegradation. These xenobiotics include DDT raw materials, the level-I cancerogenic substance, Dioxin, and atrazine, the herbicide applied most widely in the world, and the like.

| **Name of the biodegradable compound** | **Effect of the compound** | **Increment rate** | |
|---|---|---|---|
| | | **14-day dosing** | **30-day dosing** |
| Fluorobenzoate | Pesticide or medical intermediate | 229.1% | 202.8% |
| Chlorocyclohexane | Pesticide, rubber antiscorching agent, or medical intermediate | 161.8% | 122.8% |
| Chlorobenzene | Synthetic raw material of DDT; solvent of ethyecellulose and lots of resins, and other intermediates of benzene series | 161.8% | 122.8% |
| Caprolactam | Raw material of nylon plastic | 285.2% | 198.2% |
| Styrene | Important monomer of synthetic resins, ion exchange resins and synthetic rubbers and the like; styrene has been classified as category-2B carcinogen by WHO | 135.8% | 122.4% |
| Atrazine | The herbicide applied most widely in the world. Atrazine endangers mammal via endocrine disruption, genotoxicity, reproductive toxicity and the like. Atrazine has been listed a possible cancerogenic substance by Environmental Protection Agency (EPA) | 167.5% | 120.6% |
| Dioxin | Dioxin is a kind of colourless, odourless, and highly toxic liposoluble substance. Dioxin is highly toxic and exists in the air, soil and water with fine particles. Dioxin has been listed the level-I cancerogenic substance by International Agency for Research on Cancer. | 135.1% | 116.5% |
| Aminobenzoate | The purest aromatic aminocarboxylic acid | 133.1% | 112% |
| Xylene | Xylene is widely applied in the industries such as, coatings, resins, dyestuff and printing ink as a solvent. Xylene has been classified as category 3 carcinogen by WHO | 127.2% | 111.9% |

| | | | |
|---|---|---|---|
| Note: in the table above, the calculation method of the increment rate of each biodegradation-related genome is as follows: [(relative abundance of the genome with 14- or 30-day dosing/relative abundance of the genome before administration)× 100] | | | |

FIGS. 20a-20b (Y-coordinate represents the relative abundance) indicate that the composition has acceleration on a cytochrome p450 (Cyp450). Cyp450 can reduce or eliminate the bioactivity of xenobiotics, thus promoting them to be discharged from human body. From the analysis on the functional genomes of Cyp450 with the functions of Drug Metabolism (FIG. 20a) and Xenobiotics Metabolism (FIG. 20b), it can be seen that there is an accelerating trend with 14-day dosing, and the acceleration effect has statistical significances after 30 days administration. Increased Cyp450 function may be the major mechanism to accelerate the biodegradation of the above xenobiotics, including drugs, pesticides, environmental pollutants, and food additives, and the like.

FIG. 21a (X-coordinate is the relative abundance before administration; Y-coordinate is the relative abundance after administration; the dotted line represents an exponential trend) and FIG. 21b (Y-coordinate is the relative abundance) show that the composition has two-way regulation on the biosynthesis of polyketides and terpenoids. The decrease of the correlation coefficient with 30-day dosing is caused by the increase of the variation coefficient due to the sharp increase (>1100%) of the type II polyketide products. Polyketides are a category of secondary metabolites produced by bacteria, fungi, plants and animals. Due to its important bioactivity, polyketides can serve as antibiotics (e.g., erythromycin), antifungal agents (e.g., amphotericin), anti-cancer drugs (e.g., doxorubicin), immunosuppressants (e.g., rapamycin), anti-parasitic agents, and the like. Terpenoids are generic terms of a series of terpenoid compounds; the total number of the existing terpenoid compounds has exceeded 22,000 currently. Lots of terpenoid compounds have important physiological activity, and are a comparatively important category of compounds in Chinese herbal medicines, for example, tanshinones compounds, triptolide, stevioside, and rographolide, and the like. Moreover, there are further Yuanhuacin, Schisantherin and other diterpenoids ingredients with antitumor activity. These products not only have protective effects on gut microbiota, but also may provide bioactive ingredients beneficial to the host human body. What's more, as shown in the dotted line of FIG. 21a, the composition has a selective enhancement trend to less abundant genetic groups, and may promote the synthesis of the relatively scarce polyketides and terpenoids.

As shown in FIG. 22 (X-coordinate is the relative abundance before administration, and Y-coordinate is the change rate of the relative abundance after administration; the filled dots in the figure show mean values of the relative abundance of related genome; the dotted line represents an exponential trend), the composition has two-way regulation on cofactors and vitamins, and has stronger acceleration to the less abundant genetic groups. The composition is expected to promote and maintain the physiological functions of the host human body by adjusting the relatively scarce cofactors and vitamins.

As shown in FIGS. 23a-23c (x-coordinate is the relative abundance before administration, and Y-coordinate is the relative abundance after administration; the dotted line represents a linear trend), the composition has good two-way regulation on the vitamins B synthesis-related functional genomes, including vitamins B1 (left figure of FIG. 23a), B2 (right figure of FIG. 23a), B6 (left figure of FIG. 23b), folic acid (B9) (right figure of FIG. 23b), and B12 (FIG. 23c). Vitamins B need to be cooperated with each other to work. Therefore, the overall improvement of the composition on the relatively less abundant functional genomes is of important meaning to maintain and improve the physiological functions of the overall vitamins B.

FIG. 24 (Y-coordinate is the relative abundance of the lipoic acid metabolism-related genome) shows that the composition has acceleration on the cofactor lipoic acid. with 14-day dosing, the composition shows an improvement trend, and with 30-day dosing, there are remarkable statistical significances. Lipoic acid has a strong antioxidation and can relieve the risk to fatty liver and hyperlipidemia, as well as the toxicity of heavy metals on other cofactors.

As shown in FIG. 25 (X-coordinate is the relative abundance before administration, and Y-coordinate shows a change of the relative abundance after administration; the filled dots in the figure show mean values of the relative abundance of related genome; the dotted line represents an exponential trend), the composition has two-way regulation on the carbohydrate metabolism-related functional genomes (Carbohydrate Metabolism Gene Group) and shows an enhancing effect on the less abundant functional groups.

As shown in FIG. 26a (X-coordinate is the relative abundance before administration, and Y-coordinate is the change rate of the relative abundance after administration; the filled dots in the figure show mean values of the relative abundance of related genome; the dotted line represents an exponential trend), the composition has two-way regulation on the lipid metabolism. Statistical analysis indicates that there is a trend of negative correlation with 14- or with 30-day dosing. As shown in the trend represented by the dotted line, the composition has a very strong acceleration on some extremely less abundant functional genomes. Lipid includes important components (phospholipid) of cytomembrane, important physiological regulation substances (including lipid-soluble vitamins), and important intermediates of biological metabolism, and the like, which provides important material basis for vital activity. As shown in the box plot (26b), the regulation effect of the composition on tissue metabolism may be partially achieved by increasing the bile secretion.

As shown in FIG. 27 (Y-coordinate represents the relative abundance), the composition significantly improves the abundance of the linolenic acid synthesis and metabolism functional genomes (Linolenic Acid Metabolism). With 14-day administration, there is an enhancement trend, and with 30-day dosing, there are remarkable statistic differences, indicating the long-term effectiveness of the composition. Linolenic acid is a kind of ω-3-series polyenoic fatty acid, and is an important component to constitute human tissue cells, and can be synthesized, metabolized and transformed into DHA and EPA essential to human body. Linolenic acid cannot be synthesized in human body, and one of the sources is gut microbiota synthesis. The shortage of linolenic acid in human body will cause lipid metabolism disorders, resulting in immunity decrease, amnesia, fatigue, hypopsia, cardiovascular and cerebrovascular diseases, and the like. Moreover, linolenic acid is also an important cosmetic.

As shown in FIG. 28 (X-coordinate is the relative abundance before administration, and Y-coordinate is the change rate of the relative abundance after administration; the filled dots in the figure show mean values of the relative abundance of related genome; the dotted line represents an exponential trend), the technical solution of the present invention has significant two-way regulation on the functional genomes associated with amino acid synthesis and metabolism. The composition has a stronger effect on the less abundant functional genomes associated with amino acid synthesis and metabolism with 14- and 30-day dosing.

As shown in FIG. 29 (Y-coordinate is the relative abundance), the composition has certain acceleration on the relative abundance of the functional genomes associated with tryptophan metabolism. Tryptophan is a kind of precursor to neurotransmitter, serotonin. Because serotonin cannot pass through blood brain barrier by itself, peripheral blood has to be utilized such that serotonin enters the intracerebral precursor synthesis. The shortage of serotonin will cause the shortage of intracerebral serotonin. Brain serotonin is a key neurotransmitter to maintain the internal drive (appetite, sleep and sex) and emotion. The decrease of serotonin level is the major reason to cause down in spirits, depression, and manic-depressive psychosis, and may be closely related to chronic fatigue diseases, and fibromyalgia, and the like.

Besides the precursor as shown in FIG. 29, the composition has certain two-way regulation on related functional genomes to serotonin (5-HT). As shown in FIG. 30, X-coordinate of the left figure shows the relative abundance of the genetic group associated with serotonin biosynthesis before administration; Y-coordinate shows a change rate of the relative abundance after administration; the dotted line shows a linear trend. X-coordinate of the right figure shows changes of the relative abundance of the group before administration and the group with 30-day dosing, and Y-coordinate shows the relative abundance. Each line in the right figure represents a change of the relative abundance of each patient before and after administration; Pre represents the relative abundance before administration; and the right shows the increase of the relative abundance after administration. It can be seen from the table on the right of FIG. 30 that for the patient with lower relative abundance before administration (relative index is ≤16), the relative abundance is significantly improved after administration (the right of FIG. 30). Serotonin cannot pass through blood brain barrier by itself; the serotonin synthesized by gut microbiota mainly acts on end visceral organs, in particular to digestive system. Currently, the existing serotonin is related to the control of gastrointestinal peristalsis, sensitivity and secretion, and may adjust the functions of the gastrointestinal and digestive system.

Lysine is one of the essential amino acids to human and mammal, cannot be synthesized by the body itself and has to be supplemented from the outside. Gut microbiota has the genes to synthesize lysine. As shown in FIG. 31 (Y-coordinate is the relative abundance), the composition may increase the relative abundance of the functional genomes associated with lysine metabolism with 14 or 30-day dosing. Lysine is easily broken and short and thus, is called the first limiting amino acid. Lysine has important nutritional significances in the aspects of human growth and development, enhancement of body immunity, antiviral action, promotion of fat oxidation, and relief of anxiety, and the like.

As shown in FIG. 32 (X-coordinate is the relative abundance before administration, and Y-coordinate is the change rate of the relative abundance with 30-day dosing; the dotted line represents a linear trend), with 30-day dosing, the composition has two-way regulation on the functional genomes associated with short-chain fatty acid synthesis and metabolism. Short-chain fatty acids mainly include acetic acid, propionic acid and butyric acid. The composition has two-way regulation on the functional genomes associated with acetic acid and butyric acid synthesis and metabolism. The composition also has certain two-way regulation on the functional genomes associated with propionic acid synthesis and metabolism. Acetic acid is the most effective agonist to the receptor GPR43, and can promote the ileum endocrine cells, L cells to secrete glucagon-like peptide-1 (GLP-1), control inflammatory response and the like, and has multiple aspects of physiological activity. It should be emphasized that EC50 of acetic acid to GPR43 is 250 µM to 500 µM, while the mean concentration scope of acetic acid in colon cavity is 10-100 mM; calculated by the mean concentration of acetic acid, GPR43 and ligand should be always saturated. However, as shown in FIG. 32, for the individual whose relative abundance is close to 0, colon has a thick mucus layer; therefore, the continuous mucus flow and creep result in a concentration gradient of acetic acid; the acetic acid concentration in these subjects is not enough to activate the GPR43 receptor. This is the important physiological significance for the composition to enhance the relatively less abundant functional genomes. Butyric acid mainly includes the following major functions, namely, providing energy for colonic epithelial cells, inhibiting histone deacetylase (HDACs), anti-inflammatory or immune modulating function (Makki K et al., Cell Host Microbe. 2018; Koh A et al., Cell, 2016). The composition is of important meaning to solve the butyric acid shortage of these people with low relative abundance.

As shown in FIG. 33a (X-coordinate is the relative abundance before administration, and Y-coordinate is the change of the relative abundance after administration; the filled dots in the figure show mean values of the relative abundance of related genome; the dotted line represents an exponential trend), the composition has two-way regulation on the internal secretion functional genomes. There are remarkable statistical significances with 14- and 30-day dosing, in particular to the extremely less abundant melanogenesis-related functional genomes, and the relative abundance increases to 1.6-1.7 times. Melanin in skin can absorb ultraviolet ray to prevent it from penetrating into the deep part of skin and from damaging tissue cells, thus achieving a protective effect.

FIG. 33b (Y-coordinate represents the relative abundance) indicates that the composition has acceleration on biosynthesis of steroid hormones. Steroid hormones are approximately divided into five types: glucocorticoid, mineralocorticoid, as well as estrogen, progestogen and androgen of sex hormones. Glucocorticoid and mineralocorticoid relate to wide physiological systems such as, inflammation control, carbohydrate metabolism, protein metabolism, blood electrolyte level and immunoreaction. Low sex hormone will cause developmental retardation of primary/secondary sex characters of children and adolescents, abnormality of female physiological cycle and gestation period, osteoporosis caused by postmenopausal female, male and female sexual hypofunction, and climacteric syndrome, and other associated diseases.

Gut microbiota plays a decisive role to the maturity of the host human body immune system and function maintenance. As foreign bodies, gut microbiota needs to be monitored by immune system; but as a mutualistic group, gut microbiota cannot be attacked by human immune cells. Such kind of balance actually plays the role of training body's immune system to recognize harmful and innocuous exogenous substances. Gut microbiota are vividly compared to the accelerator and brake to control the host, the body's immune system by Justin L. Sonnenberg, a professor of Stanford University. At present, the most important problem is hypofunction of the brake, the light one suffers food or pollen allergy, for the serious one, immune cells will attack tissues of the body. Lipopolysaccharide (LPS) plays a crucial role to the accelerator and brake during the interaction between gut microbiota and the host body's immune system. LPS acts on the pattern recognition receptor of Toll-like receptors (TLRs). LPS promotes the development and maturity of the host body's immune system by the TLR4 receptor existing in target cells and plays the role similar to an accelerator. Meanwhile, LPS possessed by gut microbiota also plays a crucial role in adjusting the brake functions of immune system. Such a function is mainly achieved by activating the regulatory T cells to control the activity of immune system (Belkaid Y et al., Cell 2014; Tokuhara D et al., Allergology Int. 2019).The decrease of regulatory functions will cause immunologic dysfunction, and is closely related to the attack of various allergic diseases, such as food/drug/pollen body allergy, eczema; and autoimmune diseases, such as type-I diabetes, irritable bowel syndrome, inflammatory bowel disease, Crohn's disease, multiple sclerosis, rheumatoid, asthma and cancer. FIGS. 34a and 34b (Y-coordinate represents the relative abundance) indicate that the composition may significantly improve the relative abundance of LPS Biosynthesis Gene Group, and the relative abundance of functional genomes associated with LPS Biosynthesis Proteins, and may help adjusting the immunity of the host human body within the normal range.

FIG. 35 (Y-coordinate represents the relative abundance) indicates that the composition has acceleration on the extremely less abundant bacterium *Pantoea* (at the level of Genus). Currently, *Pantoea* lipopolysaccharide is orally taken or applied on skin, which may activate macrophage in vivo to have good effects on various diseases, including hyperlipidemia, diabetes, and atropine dermatitis (Nakata K et al., Food Sci Nutr.2014). The composition has acceleration on *Pantoea* to further show the regulating effect thereof on immunity, and usefulness to immune diseases.

Natural immune system is an important line of defense for human body to resist pathogen invasion. Innate immunity further has RIG-I like Receptors (RLRs) besides the above pattern recognition receptor of Toll-like Receptors (TLRs). RIG-I belongs to RLRs. Interferon is triggered for expression after detecting the 5'-triphosphoric acid double-stranded RNA (5'ppp-dsRNA) in cells derived from virus, thus inhibiting the virus infection. FIG. 36 (Y-coordinate represents the relative abundance) shows the effect of the composition on RLRs. The composition shows an improvement trend to functional genomes associated with RLRs activity with 14-day dosing, and with 30-day dosing, there are remarkable statistical significances, indicating the long-term intervention effect of the composition.

Bristol Stool Score is utilized in this present invention as an integrated index of gut microbiota diversity to analyze the effect of the composition from the system level. It can be seen from observation in human body that the composition has significant two-way regulation. As shown in FIG. 37 (Y-coordinate shows the change of the Bristol Stool Score), the types of the Bristol Stool indicate that the composition may promote the faeces to converge towards type 4. with 14-day dosing, the composition shows an improvement trend, and with 30-day dosing, there are remarkable statistical significances. The composition is proved to improve and maintain the gut microbiota diversity and have a long-term intervention effect from the phenotype level. As a kind of physical symptom, defecation also shows that the composition has two-way improvement effects on the symptoms of feces, including constipation or diarrhea.

The Cleveland Stool Score improved on the basis of the Bristol Stool Score also shows obvious improvement effects. As shown in FIG. 38 (Y-coordinate shows the change of the Modified Cleveland Stool Score), there are remarkable significant statistic differences with 14-day dosing, and with 30-day dosing, the significant therapeutic effect is persistent. The composition is further proved to improve and maintain the gut microbiota diversity and have a long-term intervention effect from the phenotype level.

To further prove the improvement effect of the composition on body functions, representative laboratory detection indexes directed to some volunteers suffering from hyperglycemia, dyslipidemia or high serum uric acid were detected. These indexes include blood lipid (serum total cholesterol, serum triglyceride, serum low density lipoprotein cholesterol, serum high density lipoprotein cholesterol), blood glucose (fasting blood-glucose and glucose tolerance test), serum uric acid, renal function indexes (serum creatinine, estimated glomerular filtration rate), and liver function indexes (serum alanine aminotransferase and serum aspartate aminotransferase).

The administration method was the same as that for the volunteers with gut microbiota detection. Administration method is briefly specified as follows: 1) the composition 1 or 4 was taken first (in this example, the subsequent test data was obtained on the condition that the composition 1 was mixed with 250-300 ml warm water and taken twice per day as the starting formula).After taking the composition, some of patients would suffer abdominal distension and even stomachache, and the symptoms of abdominal distension and stomachache would be improved or removed within two or three days generally, and then, the composition may be continuously taken. 2) If the reaction of abdominal distension and even stomachache last a week around, the composition 2 or 5 was used for replacement (in this example, the subsequent test data was obtained on the condition that the composition 2 was taken as the adjustment formula). After the patient adapted to the composition one week later, if the condition of the feces (Bristol Stool Scale) was good, the composition 2 or 5 may serve as the maintenance prescription. If the condition of feces (Bristol Stool Scale) had no obvious response, the composition 1 or 4 was taken again once the abdominal distension or stomachache was improved or removed. 3) If the condition of feces (Bristol Stool Scale) had no obvious response one week later after taking the composition 1 or 4, the composition 3 or 6 was used (in this example, the subsequent test data was obtained on the condition that the composition 3 was taken as the adjustment formula). If the condition of feces (Bristol Stool Scale) had good response, the composition 1 or 4 was taken again as the maintenance prescription. 4) The patients accompanied with constipation started to take the composition 7. When the constipation was improved after administration, the composition 1 or 4 was taken as the maintenance prescription. The administration time is one month; other indexes were detected with 30-day dosing except the glucose tolerance test was performed once respectively with 14- and 30-day dosing.

Statistical treatment: Pearson correlation coefficient was taken to the correlation, and non-correlation was excluded by paired T test. As for the intergroup control, paired T test was used to compare the change before and after administration (p < 0.05 shows significant differences).

### <Improvement effect on dyslipidemia>

### Test method:

The volunteers were divided into a normal group and an abnormal group according to the Third Report of the National Cholesterol Education Program (NCEP), NIH Publication No. 02-5215. The volunteers received blood examination before administration and with 30-day dosing. The volunteers were forbidden to take any food except drinking water after 21:00 the previous night of the examination. Serum cholesterol was subjected to conventional determination via a cholesterol oxidase method, and triglyceride was subjected to conventional determination via a GPO-PAP enzyme method in a hospital laboratory.

Test indexes of the normal and abnormal blood are shown in the table below respectively:

| Blood index | Normal group | Abnormal group |
|---|---|---|
| Total cholesterol (TC) | <5.172 mmol/L | ≥5.172 mmol/L |
| Low density lipoprotein cholesterol (LDL-C) | <2.586 mmol/L | ≥2.586 mmol/L |
| High density lipoprotein cholesterol (LDL-C) | ≥1.03 mmol/L | <1.03 mmol/L |
| Triglyceride (TG) | <1.69 mmol/L | ≥1.69 mmol/L |

### <Effect on serum total cholesterol>

Table for basic information of the 30 volunteers (16 in the normal group and 14 in the abnormal group) and data change before and after administration

| Serum total cholesterol (mmol/L) | | | | | | | | | |
|---|---|---|---|---|---|---|---|---|---|
| Normal group | | | | | Abnormal group | | | | |
| Name | Age | Gender | Before admi nistration | After admi nistration | Name | Age | Gender | Before adm inistration | After admi nistration |
| WXF | 36 | Male | 4.07 | 3.87 | DYF | 55 | Female | 5.79 | 5.03 |
| LWY | 58 | Female | 4.35 | 6.80 | ZYZ | 40 | Male | 7.31 | 5.95 |
| NJY | 48 | Male | 5.14 | 3.16 | ZHY | 57 | Female | 6.07 | 4.11 |
| WQL | 61 | Female | 5.11 | 4.04 | JZX | 71 | Female | 6.44 | 4.15 |
| wx | 46 | Male | 3.83 | 3.71 | CJY | 44 | Male | 5.72 | 4.88 |
| ZGC | 53 | Male | 4.76 | 4.70 | YWJ | 67 | Male | 5.74 | 5.29 |
| ZTH | 20 | Male | 3.72 | 3.10 | WHZ | 53 | Female | 6.55 | 6.16 |
| XDZ | 25 | Male | 4.91 | 4.11 | XMJ | 57 | Female | 6.73 | 6.6 |
| YHQ | 46 | Male | 4.62 | 6.10 | ZZM | 51 | Male | 5.86 | 5.54 |
| HCL | 62 | Male | 3.31 | 3.19 | LZZ | 58 | Male | 5.79 | 5.01 |
| XJF | 58 | Female | 3.54 | 4.28 | ZHX | 41 | Male | 5.41 | 5.43 |
| CXF | 47 | Male | 4.01 | 4.03 | WXY | 41 | Male | 9.64 | 9.33 |
| SJF | 46 | Male | 5.03 | 4.53 | WNL | 62 | Male | 5.51 | 5.61 |
| LMS | 57 | Male | 3.61 | 3.03 | LFG | 73 | Female | 5.66 | 5.87 |
| ZBK | 22 | Male | 4.83 | 4.53 | | | | | |
| CLZ | 38 | Female | 5.07 | 4.48 | | | | | |

FIG. 39a shows the effect of the composition on the total cholesterol. Y-coordinate represents the concentration of serum total cholesterol. The left figures show the effect of the composition on the abnormal total cholesterol group. The composition may obviously decrease the concentration of the serum total cholesterol, and there is a significant statistic difference (p=0.0058). The right figure shows no obvious effect of the composition on the normal total cholesterol group. The abnormal elevation of serum cholesterol is closely associated with arteriosclerosis, heart disease, stroke and other types of cardiovascular and cerebrovascular diseases (Stamler, J.et al. JAMA, 1986; Keys A. et al. Am. J. Epidemiol. 1986). Meanwhile, cholesterol is an important raw material to synthesize physiological activators such as, adrenal cortical hormone, sex hormone and other important hormones, bile acid and vitamin D demanded by fat digestion and absorption, and also an important component to constitute cytomembrane, and is an important physiological activator to maintain viscera and tissue functions of human body. The action characteristic of the composition is to specifically decrease the abnormally elevated total cholesterol, but not influence the cholesterol concentration at the normal physiological level.

### <Effect on low density lipoprotein cholesterol and high density lipoprotein cholesterol>

Serum total cholesterol is the sum of cholesterol contained in lipoprotein of blood, and mainly consists of low density lipoprotein cholesterol and high density lipoprotein cholesterol. The therapeutic effects of 30 volunteers (12 in the normal low density lipoprotein cholesterol group and 18 in the abnormal low density lipoprotein cholesterol group) were observed in this present invention. Table for basic information of the volunteers and data change before and after administration

| Low density lipoprotein cholesterol (mmol/L) | | | | | | | | | |
|---|---|---|---|---|---|---|---|---|---|
| Normal group | | | | | Abnormal group | | | | |
| Na me | Ag e | Gend er | Before administration | After administration | Na me | Ag e | Gend er | Before administra tion | After administra tion |
| WX F | 36 | Male | 2.47 | 2.32 | DY F | 55 | Fem ale | 2.88 | 2.76 |
| WQ L | 61 | Fem ale | 2.72 | 1.98 | NJ Y | 48 | Male | 3.48 | 1.96 |
| wx | 46 | Male | 2.57 | 2.04 | ZG C | 53 | Male | 2.88 | 2.74 |
| ZG C | 53 | Male | 2.88 | 2.74 | ZH Y | 57 | Fem ale | 3.24 | 2.05 |
| ZT H | 20 | Male | 2.36 | 1.74 | YH Q | 46 | Male | 3.94 | 3.57 |
| ZY Z | 40 | Male | 1.72 | 2.07 | JZX | 71 | Fem ale | 3.57 | 1.59 |
| XD Z | 25 | Male | 2.55 | 2.08 | CJY | 44 | Male | 3.32 | 2.90 |
| HC L | 62 | Male | 1.83 | 1.82 | WN L | 62 | Male | 4.26 | 3.61 |
| XJF | 58 | Fem ale | 1.65 | 2.17 | LF G | 73 | Fem ale | 3.59 | 3.48 |
| CX F | 47 | Male | 2.09 | 2.13 | ZH X | 41 | Male | 3.65 | 3.66 |
| LM S | 57 | Male | 2.31 | 1.6 | YW J | 67 | Male | 3.29 | 2.77 |
| CL Z | 38 | Fem ale | 2.33 | 1.85 | WH Z | 53 | Fem ale | 3.70 | 3.91 |
| | | | | | XM J | 57 | Fem ale | 4.53 | 3.57 |
| | | | | | ZZ M | 51 | Male | 3.22 | 3.35 |
| | | | | | SJF | 46 | Male | 3.10 | 2.91 |
| | | | | | LZ Z | 58 | Male | 4.32 | 3.18 |
| | | | | | wx Y | 41 | Male | 5.60 | 6.11 |
| | | | | | ZB K | 22 | Male | 2.99 | 2.62 |

FIG. 39b shows the effect of the composition on the low density lipoprotein cholesterol. Y-coordinate represents the concentration of serum low density lipoprotein cholesterol. The left figure shows the effect of the composition on the abnormal group. The composition may obviously decrease the concentration of the serum low density lipoprotein cholesterol, and there is a significant statistic difference (p=0.005). The right figure shows the effect of the composition on normal group. The composition also has a decrease trend on the serum low density lipoprotein cholesterol in the normal group (p=0.064).

The above result indicates that the composition has a good effect of decreasing the low density lipoprotein cholesterol. Low density lipoprotein binds to cholesterol to transport cholesterol into peripheral tissue cells. Too much low density lipoprotein in blood will form residual particles to be accumulated within the blood vessel wall under blood vessel endothelium. This is the critical first step in the formation of coronary atherosclerosis, which breaks the integrity of endothelial barrier, activates proinflammatory cytokines and chemokines, promotes the generation of reactive oxygen (ROS) (Weber, C. et al. Nat. Med. 2011), finally leading to serious diseases endangering life, such as, angina pectoris, heart stroke and cerebral stroke. Therefore, low density lipoprotein cholesterol is generally called harmful cholesterol. The composition of the present invention may significantly decrease the excessive low density lipoprotein cholesterol, and is of important meaning in preventing the occurrence of cardiovascular and cerebrovascular diseases, and their progress.

FIG. 39c shows the effect of the composition of the present invention on the high density lipoprotein cholesterol. The composition has no impact on the volunteer with a normal level of high density lipoprotein cholesterol. However, even though there is no statistical significance, a part of patients with low level of high density lipoprotein cholesterol are improved greatly.

Currently, the common clinical antilipemic agents mainly include HMG-CoA reductase inhibitors (statins drugs), cation exchange resin preparations, and cholesterol transferase inhibitors of small intestines. These therapeutics not only have the side effects of dissolving striated muscle, impairment of liver and renal functions, but also will affect the physiological functions of cholesterol with excessive amount. The composition consists of medicine-food homologous food, and has a very small possibility of side effect, and may obviously decrease the low density lipoprotein cholesterol (harmful cholesterol), specifically improve hypercholesteremia, free of affecting the physiological level of cholesterol and thus, is of great clinical significance.

### <Effect on triglyceride>

Triglyceride was tested in this present invention. Table for basic information of the 30 volunteers (9 in the normal group and 21 in the abnormal group) and data change before and after administration

| Triglyceride (mmol/L) | | | | | | | | | |
|---|---|---|---|---|---|---|---|---|---|
| Normal group | | | | | Abnormal group | | | | |
| Na me | Ag e | Gend er | Before administration | After administration | Na me | Ag e | Gend er | Before administra tion | After administra tion |
| XD Z | 25 | Male | 1.66 | 1.56 | WX F | 36 | Male | 1.82 | 1.56 |
| ZH Y | 57 | Fem ale | 1.20 | 0.85 | DY F | 55 | Fem ale | 2.44 | 0.87 |
| HC L | 62 | Male | 0.98 | 0.95 | LW Y | 58 | Fem ale | 2.18 | 1.33 |
| YW J | 67 | Male | 1.18 | 1.40 | NJ Y | 48 | Male | 2.25 | 1.35 |
| XJF | 58 | Fem ale | 1.39 | 1.21 | WQ L | 61 | Fem ale | 1.82 | 2.06 |
| WH Z | 53 | Fem ale | 1.58 | 1.76 | wx | 46 | Male | 3.28 | 2.43 |
| LZ Z | 58 | Male | 1.02 | 1.60 | ZG C | 53 | Male | 2.10 | 2.39 |
| LM S | 57 | Male | 0.96 | 0.76 | ZT H | 20 | Male | 2.07 | 1.77 |
| CL Z | 38 | Fem ale | 1.18 | 1.02 | ZY Z | 40 | Male | 8.94 | 4.21 |
| | | | | | YH Q | 46 | Male | 3.63 | 1.43 |
| | | | | | JZX | 71 | Fem ale | 3.06 | 3.36 |
| | | | | | CJY | 44 | Male | 3.07 | 1.78 |
| | | | | | WN L | 62 | Male | 1.85 | 1.45 |
| | | | | | LF G | 73 | Fem ale | 1.72 | 1.61 |
| | | | | | ZH X | 41 | Male | 2.56 | 2.42 |
| | | | | | CX F | 47 | Male | 2.91 | 1.30 |
| | | | | | XM J | 57 | Fem ale | 2.45 | 3.41 |
| | | | | | zz | 51 | Male | 3.10 | 2.11 |
| | | | | | M | | | | |
| | | | | | SJF | 46 | Male | 1.74 | 1.45 |
| | | | | | ZB K | 22 | Male | 2.33 | 1.46 |
| | | | | | wx Y | 41 | Male | 3.80 | 1.14 |

FIG. 40 shows the effect of the composition on triglyceride. Y-coordinate represents the concentration of serum triglyceride. The left figure shows the effect of the composition on the abnormal serum triglyceride group. The composition may obviously decrease the concentration of the abnormally elevated serum triglyceride, and there are significant statistic differences (p=0.0044). The right figure shows no obvious effect of the composition on the normal serum triglyceride group. The abnormal elevation of the triglyceride level is an independent risk factor to the atherosclerosis and cardiovascular and cerebrovascular diseases (Sandesara P.B., Endocr. Rev. 2019). However, the physiological level of triglyceride plays a crucial role in the aspect of energy metabolism. The composition may selectively decrease the abnormally elevated concentration of triglyceride, but is free of affecting the normal physiological level, may effectively prevent or control the occurrence and progress of ischemic heart diseases, myocardial infarction, heart failure, cerebral stroke and the like.

### <Improvement effect on fasting blood-glucose>

### Test method

Prediabetes volunteers or volunteers in the early stage of diabetes (fasting blood-glucose (FBG) >5.56 to <7.5 mmol/L) were selected as an abnormal FBG group and volunteers with FBG ≤5.56 mmol/L were selected as a normal group based on the standards of American Diabetes Association (ADA). The volunteers received blood examination before administration and with 30-day dosing. The volunteers were forbidden to take any food except drinking water after 21:00 the previous night of the examination. The FBG was determined by a glucose oxidase-peroxidase coupling method in a hospital laboratory. Table for basic information of the 32 volunteers (13 in the normal group and 19 in the abnormal group) and changes of the FBG values before and after administration

| FBG (mmol/L) | | | | | | | | | |
|---|---|---|---|---|---|---|---|---|---|
| Normal group | | | | | Abnormal group | | | | |
| Nam e | Ag e | Gend er | Before administrati on | After admini strati on | Nam e | Ag e | Gend er | Before administrati on | After admini strati on |
| DYF | 55 | Femal e | 5.06 | 5.10 | WX F | 36 | Male | 5.65 | 5.55 |
| ZTH | 20 | Male | 4.92 | 4.77 | LW Y | 58 | Femal e | 7.40 | 5.53 |
| XDZ | 25 | Male | 4.70 | 4.69 | NJY | 48 | Male | 5.63 | 4.78 |
| ZHY | 57 | Femal e | 5.15 | 5.35 | WQ L | 61 | Femal e | 5.68 | 5.41 |
| JZX | 71 | Femal e | 5.42 | 5.17 | wx | 46 | Male | 6.10 | 5.30 |
| SJB | 56 | Male | 5.20 | 5.28 | ZGC | 53 | Male | 5.76 | 6.53 |
| CXF | 47 | Male | 3.94 | 5.10 | ZYZ | 40 | Male | 5.59 | 5.54 |
| ZZ M | 51 | Male | 5.23 | 5.03 | YH Q | 46 | Male | 5.90 | 4.84 |
| ZBK | 22 | Male | 5.11 | 4.16 | WN L | 62 | Male | 5.81 | 5.49 |
| wx Y | 41 | Male | 4.68 | 5.20 | ZH X | 41 | Male | 5.96 | 5.65 |
| CYJ | 44 | Male | 5.52 | 5.60 | HCL | 62 | Male | 6.06 | 6.03 |
| XJF | 58 | Femal e | 5.41 | 6.05 | YW J | 67 | Male | 7.20 | 5.62 |
| SDL F | 46 | Male | 5.17 | 5.11 | XHJ | 57 | Femal e | 5.91 | 6.07 |
| | | | | | LZZ | 58 | Male | 5.59 | 5.93 |
| | | | | | LM S | 57 | Male | 5.99 | 5.56 |
| | | | | | XG | 74 | Male | 5.99 | 5.19 |
| | | | | | Y | | | | |
| | | | | | LNL | 74 | Male | 6.91 | 6.14 |
| | | | | | WQ F | 60 | Male | 6.85 | 5.76 |
| | | | | | LJH | 47 | Male | 6.36 | 6.26 |

FIG. 41 shows the effect of the composition on FBG. Y-coordinate represents the blood glucose concentration. The left figure shows that the composition may obviously decrease the concentration of the blood glucose for the abnormal blood glucose group, and there are significant statistic differences (p=0.0048). The right figure shows no obvious effect of the composition on the normal blood glucose group.

The present invention further utilizes glucose tolerance tests to observe the improvement effect of the composition on the insulin resistance of the 8 prediabetes or diabetes volunteers. The volunteers received blood examination before administration and with 14- and 30-day dosing. The volunteers were forbidden to take any food except drinking water after 21:00 the previous night of the examination. In the next morning, venous blood was respectively drawn at 0 min (before taking glucose), 30 min, 1 h and 2 h later after orally taking 75 g glucose solution, thus determining the blood glucose level. FIG. 42 (left) shows the effect on the glucose tolerance test with 14- and 30-day dosing. It can be seen from the figure that the composition has an inhibitory effect on the blood glucose elevation after orally taking the glucose solution, and the inhibitory effect is more obvious with 30-day dosing, indicating the long-term therapeutic effect of the composition. FIG. 42 (right) shows statistical analysis at two time points of 30 min and 1 h of the glucose tolerance test. There are significant statistic differences to the two time points of 30 min (p=0.044) and 1 h (p=0.019) with 30-day dosing. The result indicates that the composition of the present invention may improve the insulin resistance and enhance the sensibility of the body tissue cells to insulin. Insulin resistance always appears before the attack of diabetes firstly. To improve the sensibility of tissues and organs to insulin may prevent the occurrence of diabetes. After the attack of diabetes, because there is only symptomatic treatment, diabetes patients not only need to take medicine during the lifetime, but also ultimately suffer serious complications such as, cardiovascular and cerebrovascular diseases, chronic impairment of renal function, and nerve injury of nerve endings. The composition has an improvement effect on insulin resistance and may prevent the attack and progress of diabetes.

### <Improvement effect on high uric acid in serum>

### Test method

Volunteers with serum uric acid ≧420 mol/L (male) and ≧360 mol/L (female) served as an abnormal serum uric acid group, and volunteers with serum uric acid lower than the level served as a normal serum uric acid group (Liu AD et al, J Atheroscler. Theromb. 2013). The table below shows the basic information of the 28 volunteers (15 in the normal serum uric acid group and 13 in the abnormal serum uric acid group) and changes of the serum uric acid values before and after administration. The volunteers received blood examination before administration and with 30-day dosing. The volunteers were forbidden to take any food except drinking water after 21:00 the previous night of the examination. Serum uric acid was determined by coupled-enzyme assay.

| Uric acid (µmol/L) | | | | | | | | | |
|---|---|---|---|---|---|---|---|---|---|
| Normal group | | | | | Abnormal group | | | | |
| Nam e | Ag e | Gend er | Before administrati on | After administrati on | Nam e | Ag e | Gend er | Before administrati on | After admini strati on |
| DYF | 55 | Femal e | 359 | 316 | WQ L | 61 | Femal e | 380 | 353 |
| wx | 46 | Male | 363 | 387 | ZGC | 53 | Male | 520 | 498 |
| ZH Y | 57 | Femal e | 251 | 249 | ZTH | 20 | Male | 584 | 533 |
| YH Q | 46 | Male | 261 | 310 | ZYZ | 40 | Male | 456 | 456 |
| JZX | 71 | Femal e | 249 | 211 | XD Z | 25 | Male | 673 | 614 |
| LFG | 73 | Femal e | 296 | 286 | CJY | 44 | Male | 507 | 454 |
| CZH | 61 | Male | 319 | 421 | WN L | 62 | Male | 482 | 517 |
| SJB | 56 | Male | 262 | 247 | ZH X | 41 | Male | 526 | 469 |
| XMJ | 57 | Femal e | 318 | 305 | HCL | 62 | Male | 430 | 399 |
| SJF | 46 | Male | 345 | 352 | YW J | 67 | Male | 515 | 476 |
| LZZ | 58 | Male | 378 | 333 | XJF | 58 | Femal e | 424 | 375 |
| LM S | 57 | Male | 390 | 390 | ZZ M | 51 | Male | 449 | 373 |
| wx Y | 41 | Male | 352 | 369 | ZBK | 22 | Male | 519 | 496 |
| BJL | 41 | Male | 352 | 332 | | | | | |
| WS M | 61 | Male | 402 | 401 | | | | | |

FIG. 43 shows the effect of the composition on serum uric acid. Y-coordinate represents the concentration of serum uric acid. The right figure shows that the composition may obviously decrease the concentration of the blood glucose for the abnormal serum uric acid group, and there are significant statistic differences (p=0.001). The left figure shows no obvious effect of the composition on the normal serum uric acid group.

Traditionally, the diseases associated with high-level serum uric acid mainly include symptomatic high-uric acid diseases caused by uric acid crystal, such as gout, acute and chronic arthritis, uric acid-derived kidney stones, acute or chronic renal damage, and the like. In recent years, lots of epidemiological, clinical and experimental studies have indicated that the asymptomatic high-uric acid patients among the crowd have a higher proportion, and the asymptomatic high-uric acid is associated with the pathogenesis of cardiovascular and cerebrovascular diseases, central nervous disorders, diseases of immune and renal systems, erectile dysfunction of disordered sex activity, and sexual apathy and other multiple systemic diseases. The extent of harm on human health caused by these effects of soluble high uric acid is much larger than gout; therefore, high uric acid has been highly concerned in medical and science fields. Hyperuricemia has huge potential risk to human body, but uric acid is an important physiological activator and is of great significance to human health. Uric acid is a kind of powerful ROS antioxidant and peroxynitrite scavenger. Uric acid can scavenge free radicals and chelate metal ions (Frei B, et al., PNAS 1988; Glantzounis GK, et al. Curr Pharm Des 2005; Sautin YY, Johnson RJ. Nucleosides Nucleotides Nucl Acids 2008;).Half of plasma oxidation resistance of human body is from uric acid, and such kind of function is stronger than that of folic acid (Yeum KJ, et al., 2004).The physiological level of uric acid may play a protective effect on the integrity of blood vessel endothelium, and is also an important medium to human type-II immunoreaction and plays a crucial role in response to the protective antibody of vaccines (Kool M, et al. J Exp Med 2008; Kool M, et al. Immunity 2011).

The composition may not affect the normal physiological level of uric acid while controlling hyperuricemia, and is of great significance in maintaining the physiological level of uric acid, and preventing or treating diseases associated with high uric acid.

### <Improvement effect on renal functions>

### Test method

Serum creatinine and glomerular filtration rate are the basic methods to determine liver functions. The present invention utilized the detection of serum creatinine level and estimated glomerular filtration rate to prove the improvement effect of the composition on renal functions. The volunteers received blood examination before administration and with 30-day dosing. The volunteers were forbidden to take any food except drinking water after 21:00 the previous night of the examination. In the next morning, venous blood was drawn to conventionally determine the serum creatinine level by a creatinine enzyme method. The table below shows the basic information of the 23 volunteers and changes of the serum creatinine level before and after administration.

| Name | Age | Gender | Creatinine (□ mol/L) | |
|---|---|---|---|---|
| | | | Before administration | After administration |
| WQL | 61 | Female | 47 | 53 |
| DYF | 55 | Female | 50 | 54 |
| XMJ | 57 | Female | 53 | 58 |
| LFG | 73 | Female | 60 | 55 |
| YHQ | 46 | Male | 62 | 59 |
| JZX | 71 | Female | 62 | 61 |
| BJL | 41 | Male | 62 | 65 |
| CZH | 61 | Male | 64 | 70 |
| YWJ | 67 | Male | 66 | 54 |
| ZHY | 57 | Female | 67 | 65 |
| ZTH | 20 | Male | 74 | 67 |
| SJB | 56 | Male | 74 | 59 |
| WXY | 41 | Male | 74 | 66 |
| WNL | 62 | Male | 76 | 75 |
| SJF | 46 | Male | 76 | 74 |
| HCL | 62 | Male | 79 | 67 |
| ZBK | 22 | Male | 81 | 66 |
| ZYZ | 40 | Male | 86 | 75 |
| CJY | 44 | Male | 87 | 79 |
| WXF | 36 | Male | 89 | 82 |
| YZP | 51 | Male | 92 | 77 |
| ZGC | 53 | Male | 96 | 89 |
| WSM | 61 | Male | 129 | 106 |

FIG. 44 shows correlation analysis on the serum creatinine level before administration and concentration change of serum creatinine after administration Y-coordinate represents the concentration of serum creatinine. The composition may obviously decrease the serum creatinine, and there are significant statistic differences (p=0.0014). Serum creatinine is one of the best indexes to reflect glomerular filtration function. The decrease of the composition on the concentration of serum creatinine reflects the protective effect of the composition on renal functions. Hyperuricemia, hyperglycemia and dyslipidemia will cause impairment of renal function. The protective effect of the composition on renal functions may be an integrated reaction to improve hyperuricemia, hyperglycemia and dyslipidemia.

FIG. 45 shows correlation analysis on the change rate of serum creatinine after administration with the serum creatinine level before administration. Y-coordinate represents the change rate of serum creatinine concentration after administration; X-coordinate represents the serum creatinine level before administration; the filled dots in the figure show detection data of each patient; the curve shows a linear trend. The decrease degree of the composition on the serum creatinine level is strongly correlated to the serum creatinine level before administration (Correl R=-0.59367); there are significant statistic differences (p=0.000544). The result indicates that the composition may have a stronger ability to promote creatinine to be discharged from the kidney for the volunteer with a higher creatinine level before administration. Such kind of action form is unique and may be related to the two-way regulation thereof on gut microbiota.

FIG. 46 shows effects of the composition on a part of volunteers with low serum creatinine level (less than 60 mol/L) before administration. The composition may increase the serum creatinine of these volunteers on the contrary, and there are significant statistic differences (p=0.013). Basic serum creatinine is not only an optimal test index for renal function, but also can reflect the basic metabolism of muscle. Low level of creatinine is mainly reflected in consenescence conditions such as, muscle recession, emaciation and liver function damage. The action may be from the enhancement of the composition, and the two-way regulation on gut microbiota. Such an action characteristic shows the adjustment characteristics of the composition on human physiological functions more.

### Effect on the estimated glomerular filtration rate (eGFR)

### Estimation method

eGFR was calculated by the China's improved approach based on the measured value of the above serum creatinine. Volunteers with eGFR<90 mi/L served as an abnormal glomerular filtration rate group. The table below shows basic information of the volunteers and data change after administration.

| Estimated glomerular filtration rate (eGFR) (ml/min) | | | | | | | | | |
|---|---|---|---|---|---|---|---|---|---|
| Normal group | | | | | Abnormal group | | | | |
| Nam e | Ag e | Gend er | Before administrati on | After admini strati on | Nam e | Ag e | Gend er | After admini strati on | After admini strati on |
| DYF | 55 | Femal e | 105.0 | 102.3 | ZGC | 53 | Male | 77.4 | 84.9 |
| WQ L | 61 | Femal e | 102.7 | 98.7 | ZH Y | 57 | Femal e | 87.6 | 90.9 |
| WX F | 36 | Male | 95.6 | 105.6 | JZX | 71 | Femal e | 87.4 | 88.0 |
| wx Y | 41 | Male | 108.5 | 122.6 | CJY | 44 | Male | 87.9 | 97.8 |
| ZTH | 20 | Male | 126.2 | 132.4 | LFG | 73 | Femal e | 87.1 | 89.0 |
| ZYZ | 40 | Male | 96.9 | 109.8 | SJF | 46 | Male | 89.0 | 105.2 |
| YH Q | 46 | Male | 113.1 | 116.2 | YZP | 51 | Male | 82.7 | 105.0 |
| WN L | 62 | Male | 96.4 | 93.5 | WS M | 61 | Male | 51.2 | 64.9 |
| CZH | 61 | Male | 100.5 | 96.8 | | | | | |
| HCL | 62 | Male | 91.5 | 97.5 | | | | | |
| YW J | 67 | Male | 95.1 | 102.0 | | | | | |
| SJB | 56 | Male | 98.0 | 107.0 | | | | | |
| XMJ | 57 | Femal e | 101.5 | 98.8 | | | | | |
| BJL | 41 | Male | 117.1 | 114.9 | | | | | |
| ZBK | 22 | Male | 118.2 | 130.5 | | | | | |

FIG. 47 shows the change of eGFR after administration. Y-coordinate represents eGFR. The left figure shows the improvement effect of the composition on the abnormal glomerular filtration rate group; and there are significant statistic differences (p=0.010). The right figure shows the effect of the composition on the normal glomerular filtration rate group; and there are also significant statistic differences (p=0.031). eGFR may reflect the renal function level better than serum creatinine, and is the way to measure the renal function level and determine the stage of renal diseases. When eGFR value is 60-89 ml/min, it indicates that kidney has slight hypofunction. The treatment started in such stage is the most promising to improve or control the further hypofunction of renal functions. However, there is no good drug at present. The composition shows a good improvement effect on the patients in this stage. When the eGFR value is 90 or more, even though the production function is in the normal scope, diseases associated with lifestyle such as, hyperglycemia, hyperlipidemia, hypertension and obesity are potential risk factors to the impairment of renal function once attack. The composition may relieve the risk of the impairment of renal function to such kind of patients.

### <Improvement effect on liver functions>

### Test method

Hepatocellular damage index threshold values obtained by the studies on non-alcoholic fatty liver diseases were used in this present invention (Bedogni G et al. Hepatology 2005). Volunteers with alanine aminotransferase or aspartate aminotransferase >30U/L served as objects to observe the changes before administration and with 30-day dosing. The volunteers were forbidden to take any food except drinking water after 21:00 the previous night of the examination. In the next morning, venous blood was drawn to determine the activity of alanine aminotransferase or aspartate aminotransferase by a velocity method. The table below shows the basic information of the 10 volunteers and activity changes of the alanine aminotransferase or aspartate aminotransferase before and after administration.

| Name | Age | Gender | Alanine aminotransferase (U/L) | | Aspartate aminotransferase (U/L) | |
|---|---|---|---|---|---|---|
| | | | Before administration | After administration | Before administration | After administration |
| ZTH | 20 | Male | 35 | 30 | 24 | 23 |
| ZYZ | 40 | Male | 65 | 27 | 30 | 17 |
| XDZ | 25 | Male | 59 | 53 | 35 | 32 |
| YHQ | 46 | Male | 30 | 16 | 53 | 17 |
| JZX | 71 | Female | 32 | 29 | 34 | 31 |
| HCL | 62 | Male | 46 | 59 | 31 | 34 |
| XJF | 58 | Female | 66 | 28 | 64 | 37 |
| SJB | 56 | Male | 32 | 31 | 24 | 24 |
| CXF | 47 | Male | 44 | 21 | 30 | 22 |
| JJ | 56 | Female | 42 | 19 | 30 | 22 |

FIG. 48 (left) shows the improvement effect of the composition on alanine transaminase; FIG. 48 (right) shows the improvement effect of the composition on aspartate aminotransferase (Y-coordinate represents a liver function unit). The composition has significant improvement effects (p=0.027, p=0.039) statistically to alanine transaminase and aspartate aminotransferase to prove that the composition has a protective effect on hepatic cells in the risk state of damage. The elevation of blood glucose and abnormal blood lipid will affect the functions of multiple organs. Liver, as an important viscera of the general metabolic function, usually suffers dysfunction in case of hyperglycemia or abnormal blood lipid. The protective effect of the composition on liver functions may be partially from the decrease of blood glucose and blood lipid.

## Claims

1. A composition functioning to improve personalized gut microbiota diversity, comprising ingredients for strengthening spleen and invigorating Qi, ingredients for clearing damp and promoting the circulation of blood, and intestinal bacteria substrates.

2. The composition functioning to improve personalized gut microbiota diversity according to claim 1, wherein the ingredients for strengthening spleen and invigorating Qi comprise one or more of *Panax ginseng,* an extract of *Panax ginseng, Panax quiquefolium,* and an extract of *Panax quiquefolium.*

3. The composition functioning to improve personalized gut microbiota diversity according to claim 1, wherein the ingredients for clearing damp and promoting the circulation of blood comprise an ingredient for clearing damp and an ingredient for promoting the circulation of blood, wherein the ingredient for clearing damp comprises one of or a mixture of more of *Wolfiporia cocos* and an extract of *Wolfiporia cocos;* and the ingredient for invigorating the circulation of blood comprises one of or a mixture of more of nattokinase, natto, and an extract of natto.

4. The composition functioning to improve personalized gut microbiota diversity according to claim 1, wherein the intestinal bacteria substrates comprise a water-soluble dietary fiber, a water-insoluble dietary fiber, or any combination thereof.

5. The composition functioning to improve personalized gut microbiota diversity according to claim 4, wherein the water-soluble dietary fiber comprises one or more of konjaku flour, glucomannan, crude laminarin, pectin, alginic acid, glucan, guar gum, inulin and *Pericarpium citri reticulatae*; or one or more of food ingredients or medicinal materials containing the above ingredients.

6. The composition functioning to improve personalized gut microbiota diversity according to claim 4, wherein the water-insoluble dietary fiber comprises one or more of cellulose, hemicellulose, and lignin.

7. The composition functioning to improve personalized gut microbiota diversity according to claim 1, further comprising one or more of *Siraitia grosvenorii,* an extract of *Siraitia grosvenorii,* almond, an extract of almond, perilla seed, and an extract of perilla seed.

8. The composition functioning to improve personalized gut microbiota diversity according to any one of claims 1-7, wherein the ingredients for strengthening spleen and invigorating Qi comprise one or more of *Panax ginseng,* the extract of *Panax ginseng, Panax quiquefolium,* and the extract of *Panax quiquefolium;* the ingredients for clearing damp and promoting the circulation of blood comprise an ingredient for clearing damp and an ingredient for promoting the circulation of blood, wherein the ingredient for clearing damp comprises one of or a mixture of more of *Wolfiporia cocos* and an extract of *Wolfiporia cocos;* and the ingredient for promoting the circulation of blood comprises one of or a mixture of more of nattokinase, natto, and an extract of natto; a daily dose mainly consists of:
| | |
|---|---|
| One of or a mixture of more of *Panax ginseng,* the extract of *Panax ginseng, Panax quiquefolium,* and an extract of *Panax quiquefolium* | 0.001-2 g |
| One of or a mixture of more of *Wolfiporia cocos* and the extract of *Wolfiporia cocos* | 0.001-5 g |
| One of or a mixture of more of nattokinase, natto, and the extract of natto | 500-4000 Fu |
| Water-soluble dietary fiber by total weight of the composition | 10%-70% |
| Water-insoluble dietary fiber by total weight of the composition | 10%-70% |
wherein an adding amount of the natto or the extract of natto is calculated by the fibrin degradation unit of the nattokinase contained therein.

9. The composition functioning to improve personalized gut microbiota diversity according to claim 8, wherein the daily dose mainly consists of:
| | | |
|---|---|---|
| Ingredients for strengthening spleen and invigorating Qi | One of or a mixture of more of *Panax ginseng* and *Panax quiquefolium* | 0.01-2 g |
| | Or, one of or a mixture of more of the extract of *Panax ginseng* and the extract of *Panax quiquefolium* | 0.001-0.2 g |
| Ingredients for clearing damp and promoting the | One of or a mixture of more of *Wolfiporia cocos* and the extract of *Wolfiporia cocos* | 0.001-5 g |
| circulation of blood | One of or a mixture of more of nattokinase, natto, and the extract of natto | 500-4000 Fu |
| Water-soluble dietary fiber by total weight of the composition | | 10%-70% |
| Water-insoluble dietary fiber by total weight of the composition | | 10%-70% |
wherein an adding amount of the natto or the extract of natto is calculated by the fibrin degradation unit of the nattokinase contained therein.

10. The composition functioning to improve personalized gut microbiota diversity according to claim 8, further comprising 0.1-3 g *Siraitia grosvenorii* or 0.001-0.5 g extract of *Siraitia grosvenorii;* or simultaneously or separately comprising 0.25-1 g) almond and/or 0.25-1 g perilla seeds.

11. The composition functioning to improve personalized gut microbiota diversity according to any one of claims 1-10, wherein the water-soluble dietary fiber accounts for 20%-60% by the total dose of the formula; the water-insoluble dietary fiber accounts for 20%-60% by the total dose of the formula; calculated by the daily dose, the water-soluble dietary fiber or the water-insoluble dietary fiber has an adding amount of 1-10 g.

12. The composition functioning to improve personalized gut microbiota diversity according to any one of claims 1-10, wherein calculated by the daily dose, the composition comprises the following ingredients:
| | |
|---|---|
| One of or a mixture of more of *Panax ginseng* or *Panax quiquefolium* or one of or a mixture of more of the extract of *Panax ginseng* or the extract of *Panax quiquefolium* | 0.1-1 g |
| | 0.01-0.04 g |
| *Wolfiporia cocos* | 0.5-4 g |
| Nattokinase | 500-4000 Fu |
| Guar gum | 0.05-0.4 g |
| Alginic acid | 0.05-0.4 g |
| Crude laminarin | 0.05-0.4 g |
| *Pericarpium citri reticulatae* | 0.05-0.4 g |
| Glucomannan | 0.1-0.8 g |
| *Psyllium* seed husk | 0.25-2 g |
| *Siraitia grosvenorii* | 0.35-1.4 g. |

13. An application of the composition of any one of claims 1-12 in the preparation of a medicament, a health-care product or food functioning to improve personalized gut microbiota diversity, or in prevention, or treatment or adjunctive treatment of a gut microbiota imbalance-related disease.

14. The application according to claim 13, wherein the medicament is a medicament for prevention, or treatment or adjunctive treatment of an gut microbiota imbalance-related disease; the health-care product or food is a health-care product or food having a function of improving gut microbiota imbalance.

15. The application according to claim 14, wherein the gut microbiota imbalance comprises decreased gut microbiota diversity.

16. The application according to claim 14, wherein the gut microbiota imbalance comprises relatively strong strains and functions of dominant gut microbiota, and relatively less abundant strains and functions of gut microbiota.

17. The application according to claim 14, wherein the gut microbiota imbalance-related disease comprises metabolic diseases, immune system diseases, dermatological diseases, mental/neurogenic diseases and digestive diseases which are associated with gut microbiota imbalance, as well as a low sex hormone-related disease.

18. The application according to claim 17, wherein the metabolic dysfunction disease comprises prediabetes, type-II diabetes, obesity, cardiovascular diseases, cerebrovascular diseases, dyslipidemia, hypertension, hyperuricemia and renal dysfunction.

19. The application according to claim 17, wherein the immune system diseases comprises food/drug/pollen allergy, eczema, type-I diabetes, irritable bowel syndrome, inflammatory bowel disease, Crohn's disease, multiple sclerosis, rheumatoid, asthma and cancer.

20. The application according to claim 17, wherein the dermatological diseases comprises atopic dermatitis, psoriasis, eczema, acne, pachylosis and skin aging which are associated with gut microbiota imbalance.

21. The application according to claim 17, wherein the mental/neurogenic disease comprises depression, bipolar disorder, autism, schizophrenia, insomnia, Parkinson's disease, Alzheimer's disease and fibromyalgia.

22. The application according to claim 17, wherein the digestive system disease comprises constipation, diarrhea, fatty liver and liver cirrhosis.

23. The application according to claim 17, wherein the low sex hormone-related disease comprises developmental retardation of primary/secondary sex characters of children and adolescents, abnormality and disease associated with female physiological cycle and gestation period, osteoporosis caused by estrogen deficiency of postmenopausal female, male and female sexual hypofunction, and climacteric syndrome.

24. The application according to claim 13, wherein the medicament, health-care product or food is a medicament, health-care product or food having a function of enhancing body biodegradation to an exogenous toxic substance; the exogenous toxic substance comprises one or more of pesticides, herbicides, food additives, plastic residues, rubber residues, environmental pollutants, heavy metals, and other hazardous chemical substances.

25. The application according to claim 24, wherein the exogenous toxic substance comprises one or more of halobenzoic acid, halocyclohexane, halobenzene, caprolactam, 2-chloro-4-ethylamino-6-isopropylamino-1,3,5-triazine, dioxin, aminobenzoate and xylene.
